# EUROPEAN PATENT APPLICATION

(11) **EP 2 790 017 A1**
(43) Date of publication of application: **15.10.2014**
(21) Application number: 13305469.2
(22) Date of filing: 11.04.2013
(51) Int. Cl.: G01N 33/50, G01N 33/569

(54) **Methods to obtain a novel class of gram negative bacteria antibiotics which target an unknown cell division associated protein LOP1**

(71) Applicant: INSTITUT PASTEUR, 75015 Paris (FR); INSTITUT NATIONAL DE LA SANTE ET DE LA RECHERCHE MEDICALE (INSERM), 75013 Paris (FR)
(72) Inventor: Marteyn, Benoit, 75013 Paris (FR); Sansonetti, Philippe, 75014 Paris (FR)
(74) Representative: Leblois-Préhaud, Hélène Marthe Georgette

(57) **Abstract**

The present invention relates to methods to identify substances which affect bacterial cell division by interfering with the function of LOP1, comprising bringing into contact a purified protein selected from the group: FtsZ, FtsQ, FtsL, FtsI and FtsN; with purified LOP1 protein and then assaying the formation of complexes between LOP1 and the selected purified protein in the presence and absence of a substance to be tested and then selecting substances from step b) which affect the formation of complexes when present. The present invention also relates to inhibitors of the activity and expression of LOP1

## Description

The present invention relates to methods to identify antibiotics which affect a previously unknown essential factor in gram negative bacterial cell division Loopine 1 (LOP1). The present invention also relates to materials which affect the expression or activity of LOP1, such as anti-LOP1 antibodies or aptamers and iRNA or derivatives of LOP1.

The life and survival of all organisms is dependent on their ability to divide. Understanding the cell division process requires accurate knowledge of cell enlargement, location and timing of the division, which include complex biological processes and requires careful coordination to initiate and complete this event. This process is particularly a challenge for prokaryotic cells, which are devoid of organelles or centrosomes. Until now, more is known about the start of this process than the end. FtsZ was the first protein identified to localise at the midcell furrow during bacterial division (Bi and Lutkenhaus, 1991). FtsZ is a GTPase functionally and structurally homologous to eucaryotic tubulin. FtsZ polymerises at midcell forming a large ring-like network at the cell membrane, known as the Z-ring (Bi and Lutkenhaus, 1991; Chen et al., 1999). The formation and subsequent constriction of the Z-ring leads to the recruitment of other essential proteins forming a mature divisome. This mature complex contains all the proteins recruited for lateral cell wall biosynthesis and completion of septation.

The divisome is composed of at least 9 essential proteins each of which plays a direct role in the cell division process (ZipA, FtsA, FtsK, FtsQ, FtsL, FtsB, FtsW, FtsI and FtsN) (reviewed in (de Boer, 2010)). The functions of these proteins and the dynamics of their interaction in the division cycle are far from understood. In addition to the essential division factors, there are an increasing number of accessory proteins recruited to the divisome, some of which are conditionally essential depending upon the environment the bacteria are replicating in. In *E coli,* midcell localization of the Z-ring is mediated by the oscillating Min system (MinC, MinD and MinE) ((Raskin and de Boer, 1997) and reviewed in (Rothfield et al., 2005; de Boer, 2010). Thus, septation of the two daughter-cell is mediated through FtsZ positioning. The formation of which dictates the position of the mature divisome and thus the site of septation. (Adams and Errington, 2009).

FtsZ assembly into filaments depends on GTP binding but not hydrolysis (Mukherjee and Lutkenhaus, 1994). Compared to tubulin FtsZ polymers contain a FtsZ-GTP and FtsZ-GDP mixture (Oliva et al., 2004) (Bi and Lutkenhaus, 1991; Romberg and Mitchison, 2004). The highly dynamic nature of FtsZ polymers is mediated by GTP hydrolysis leading to the disassembly and reduction in length of the protofilaments (Bi and Lutkenhaus, 1991; Mukherjee and Lutkenhaus, 1998; Chen et al., 1999) (Stricker et al., 2002; de Boer, 2010). In eukaryotic cells, MAPs (microtubule associated proteins) control the stability, bundling and disassembly of tubulin polymers. To date only FtsZ polymerization inhibitors have been identified, including SulA (Bi and Lutkenhaus, 1991; Trusca et al., 1998) (Bi and Lutkenhaus, 1991; Mukherjee et al., 1998; Chen et al., 1999) and MinC (Hu et al., 1999; de Boer, 2010). ZipA was shown to protect FtsZ from ClpXP-degradation (Raskin and de Boer, 1997; Pazos et al., 2012). However, the mechanisms of constriction of the Z-ring and its control remains unknown.

The inventors have now elucidated a key aspect of cell division in gram-negative bacteria and in relevant part have identified a novel cell division protein called Loopin 1 (Lop1), that is conserved among Gram-negative bacteria and that plays a key role in the disassembly of the Z ring at the final stages of cell septation. Lop1 is an ATP-dependent serine protease that is transiently recruited to the Z-ring at the onset of the mother cell constriction to trigger the ATP-dependent proteolysis of FtsZ and Z-ring constriction leading to the physical separation of the two daughter cells.

In accordance with a first aspect of the present invention there is provided a method to identify substances which affect bacterial cell division by interfering with the function of LOP1, comprising the steps:
a) Bringing into contact a purified protein selected from the group: FtsZ, FtsQ, FtsL, FtsI and FtsN; with purified LOP1 protein;
b) Assaying the formation of complexes between LOP 1 and the selected other protein in the presence and absence of a substance to be tested;
c) Selecting substances from step b) which affect the formation of complexes when present.

The inventors have shown for the first time that LOP1 plays an essential role in cell division in gram-negative bacteria. The inventors have shown via disrupting the function or expression of the LOP1 gene that the resulting bacteria show very aberrant cell division phenotypes. The inventors have characterised the parts of the divisome with which LOP1 interacts namely FtsZ, FtsQ, FtsL, FtsI and FtsN.

According to this aspect of the present invention there is provided a method to look for substances which affect the interaction of LOP1 with one or more of these portions of the divisome. Examples of substances include inorganic or organic chemical molecules, as well as substances such as antibodies or aptamers which specifically bind to LOP 1 or one of its partners.

The formation of complexes between LOP1 and one or more its target proteins can be monitored via a number of different means, for instance the detection of direct protein-protein interactions using conventional direct observational means such as spectroscopy or via indirect measurements such Surface plasmon resonance (SPR). In addition one or both of the proteins maybe labelled using a tag and then measurements made of their interaction using Fluorescence resonance energy transfer (FRET) or resonance energy transfer (RET). Such assay methods also include radioimmunoassays, competitive-binding assays, co-immunoprecipitation, pulldown assay, Western Blot analysis, antibody sandwich assays, antibody detection and ELISA assays.

In addition means of monitoring the formation of complexes between LOP1 and one its partners can also be made based upon the alteration the partner as a consequence of its interaction with LOP1. For instance the inventors have shown that FtsZ polymers are degraded by LOP1 and more specifically the self-proteolysed fragment LOP1Δ₁₋₅₉ of LOP1. In accordance with this aspect of the invention therefore the measurement of complex formation may also be made by determining the degradation of for instance FtsZ. An example of a substance which would inhibit this degradation is benzamidin, a serine protease inhibitor shown in the examples below by the inventors to prevent FtsZ polymer degradation when in the presence of LOP1Δ₁₋₅₉.

In accordance with this aspect of the present invention there is provided a method to screen for a substance affecting cell division in a gram negative bacteria comprising the steps:
a) Incubating FtsZ polymers with LOP1 in the presence and absence of a substance to be tested;
b) Assaying the degradation of said FtsZ polymers in the presence and absence of a substance to be tested;
c) Selecting substances which when present in step b) affect the degradation of FtsZ polymers.

The inventors have carefully characterised the effect of LOP1 upon its partner FtsZ, which is that it is transiently recruited to the Z-ring at the onset of the mother cell constriction to trigger the ATP-dependent proteolysis of FtsZ and Z-ring constriction leading to the physical separation of the two daughter cells.

Substances which affect either the recruitment of LOP 1 to the Z-ring and/or its proteolytic activity upon the FtsZ polymers comprised in the Z-ring, would affect cell division and hence represent a new class of antibiotic.

In accordance with this aspect of the present invention the inventors have developed a novel fluorescence based assay which measures FtsZ polymer proteolysis by monitoring the fluorescence of a solution comprising a FtsZ/FtsZ-GFP mixture. Polymerisation of the FtsZ/FtsZ-GFP mixture leads to an increase of the solution fluorescence. The further addition of LOP1Δ₁₋₅₉ leads to a degradation of the FtsZ/FtsZ-GFP polymers and hence a reduction in fluorescence.In accordance with the present invention either full length LOP1 (SEQ ID NO: 25) or a truncated version LOP1Δ₁₋₅₉ (SEQ ID NO: 26) comprising the N-terminal portion may be used in the methods according to the present Patent Application.

The inventors have shown that LOP1 undergoes ATP-dependent self-proteolysis leading to an active N-terminal portion comprising 59 residues which has serine protease activity, this active fragment is referred to as LOP1Δ₁₋₅₉ (SEQ ID NO: 26).

In accordance with the present invention there is provided a further method to identify substances which affect either the auto-proteolysis and/or ATP hydrolysis of LOP1, comprising the steps:
a) Incubating LOP1 with a substance to be tested in the presence and absence of ATP;
b) Monitoring the formation of LOP1Δ₁₋₅₉;
c) Selecting substances which when present in step b) decrease the formation of LOP1Δ₁₋₅₉

In accordance with a further aspect of the present invention there is provided a method to identify substances which affect the serine protease activity of LOP1Δ₁₋₅₉, comprising the steps:
a) Incubating LOP1Δ₁₋₅₉ with a target protein comprising at least one serine protease target site, in the presence and absence of a substance to be tested;
b) Monitoring the cleavage of the target protein;
c) Selecting substances which when present in step b) decrease the cleavage of said target protein.

The serine protease activity of LOP1Δ₁₋₅₉ on FtsZ polymers in the Z-ring is a mechanism is associated with the constriction of the Z-ring and hence cell division. Substances which affect the serine protease activity of LOP1Δ₁₋₅₉ therefore will disrupt cell division and hence represent a new class of antibiotic.

The detection and quantification of serine protease activity is well known in the art and several established methods exist such as Colorimetric or Fluorescent Detection methods (Sigma PCO100& PFO100, Twining, 1984).

In accordance with a preferred embodiment of the present invention the target protein is a FtsZ polymer.

In accordance with a further aspect of the present invention there is provided an inhibitor of the activity or expression of LOP1 or an active derivative thereof selected from the group antibodies, aptamers, antisense RNA or antisense DNA molecules or ribozymes.

Given the essential role of LOP1 in cell division in gram negative bacteria an inhibitor of the activity of LOP1 such as an anti-LOP1 antibody or aptamer; or an inhibitor of the expression of LOP1 such as an interference polynucleotide such as iRNA or siRNA, iDNA, siRNA or ribozymes; would be useful in interfering with the division of bacteria and hence represents a new class of antibiotic or research material.

In addition to the listed materials, any other means of affecting the activity or expression of LOP1 are also comprised within the present invention for instance alternative antibody replacement technologies such as nanofitins or alternative si-nucleotide systems such as shRNA.

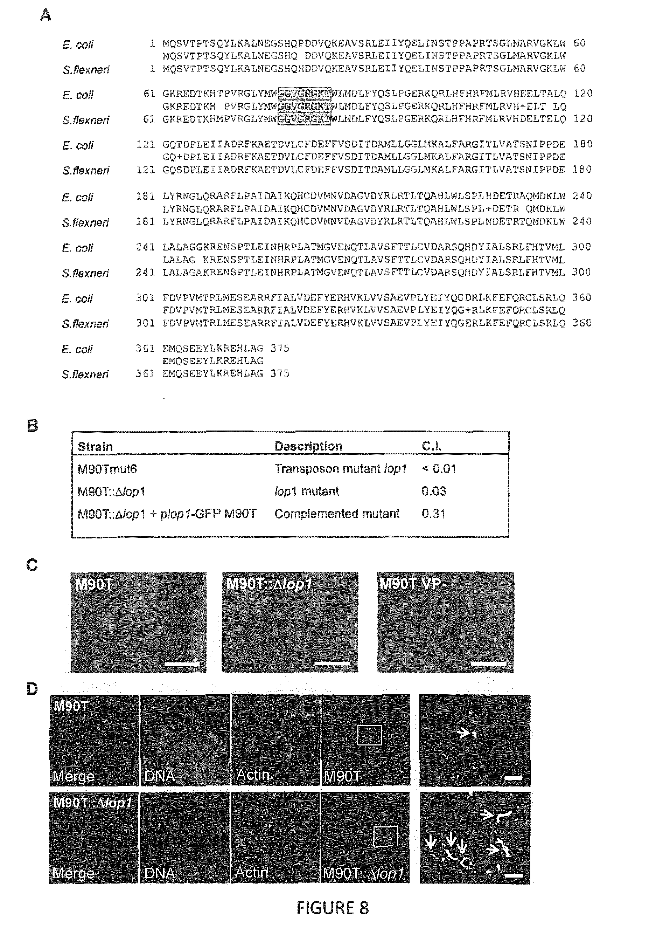

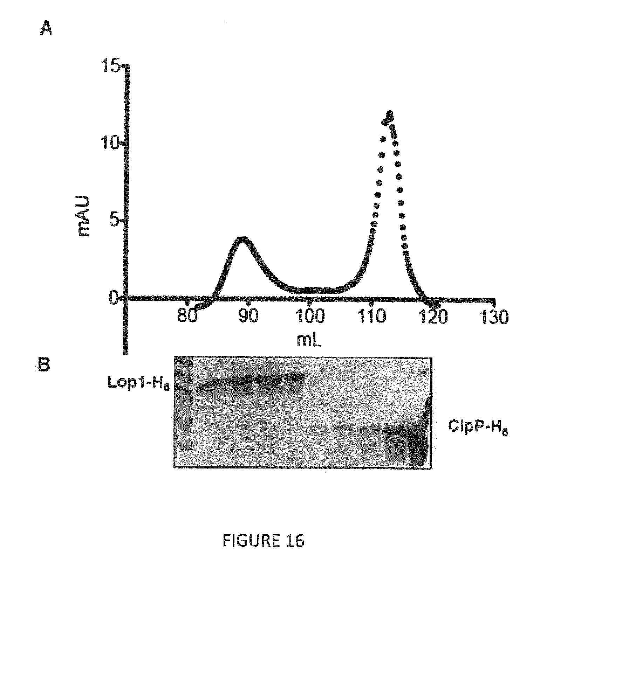

For a better understanding of the invention and to show how the same may be carried into effect, there will now be shown by way of example only, specific embodiments, methods and processes according to the present invention with reference to the accompanying drawings in which:

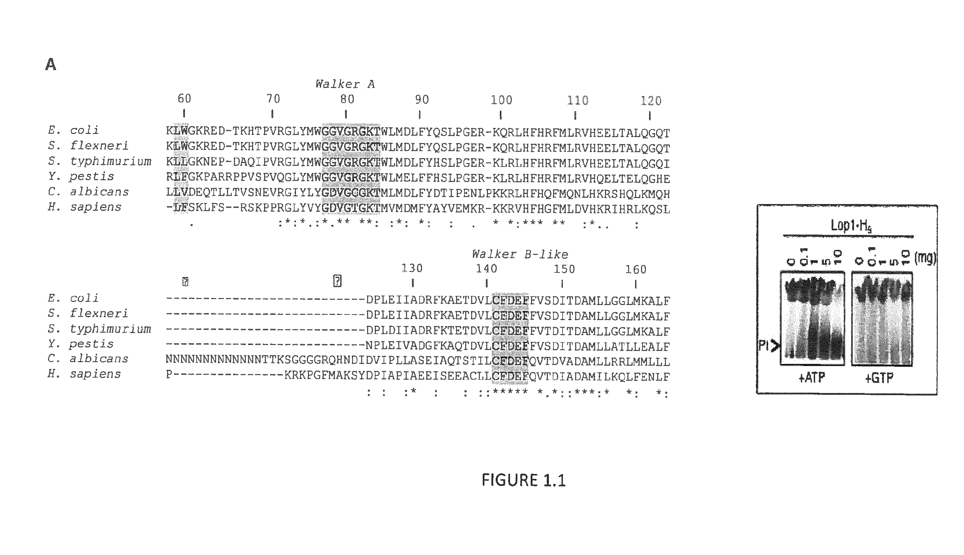

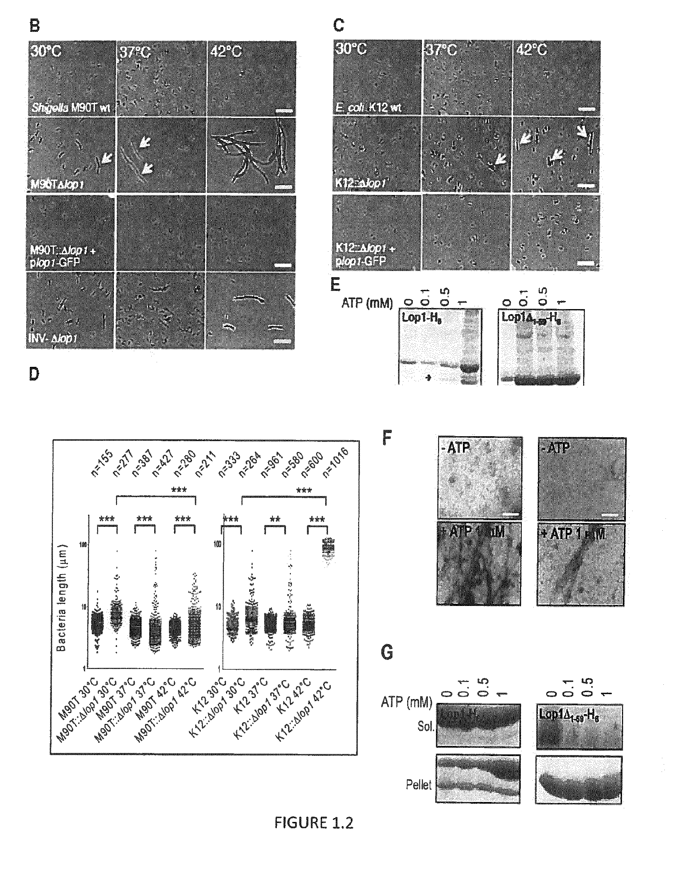

**Figure 1****. *Lop1* encodes for an ATPase. *lop1* inactivation leads to a temperature-dependent elongated phenotype of *E. coli* K12 and *Shigella flexneri* (M90T).**
   (A) Partial primary sequence alignment of Lop1 proteins from different species. Alignment was made using the ClustalW software and identification of putative Walker A and Walker B consensus sequences are highlighted. Alignment shows in Lop1 homologues identified in *E. coli* K12 MG1655 (B3232, YhcM), *Shigella flexneri 5A* M90T (S3487), *Salmonella typhi* (STY3526), *Yersinia pestis* (YPO3564), *Candida albicans* (AFG11) *and Homo sapiens* (LACE1). Sequence ID, % of homology and % of identity and P-loop sequence are detailed in Table 3. Lop1 ATPase activity was demonstrated using a silica layer chromatography technic (see Figure 9B for Lop1_{K84A} control).
   **(B** and **C)** Temperature-dependent phenotypic analysis of *E. coli* (K12) and *Shigella* (M90T) wild-type and Δ*lop1* mutants (K12::*Δlop1* (*b3232, yhcM*)*,* M90T*::Δlop1 (s3487)),* avirulent M90T VP*-*::*Δlop1* and the complemented strains *(E. coli* K12::Δ*lop1*/*plop1*-GFP, M90T*::Δlop1*/p*lop1-*GFP)*.* Bacteria were grown in a rich liquid media at the indicated temperature until an OD₆₀₀=0.5 was reached. Scale bars are 10 µm.
   **(D)** Bacteria length measurement performed on each strains and conditions described in 1B and 1C panels (see also Figure 8D) using the MicrobeTracker software (Sliusarenko et al., 2011). Three independent bacterial cultures were imaged for each strain in each growth condition. 'n' indicates the total number of measured bacteria per condition. *** indicates statistical significance between highlighted conditions, < 0.001 and ** indicates *p* < 0.01 (Student's T test).
   **(E)** Protein stability assay. *E. coli* K12 Lop1-H₆ or Lop1Δ₁₋₅₉-H₆ (80µg) were incubated at 37°C during 10 min in a TrisHCl 50 mM pH=7.4 buffer containing 5mM MgCl₂ in the presence of indicated concentration of ATP. SDS-PAGE gel with Coomassie staining.
   **(F)** Ttransmission electron microscopy (TEM) analysis of Lop1-H₆ or Lop1A₁₋₅₉-H₆ polymer formation on samples described in (E) in the absence or presence of ATP (1mM). Samples were stained with 1% uranyl acetate. Bars are 200 nm.
   **(G)** The solubility of Lop1-H₆ was assessed as described in (C) with 200 µg protein after ultracentrifugation (80000 rpm, 11min, 4°C). SDS-PAGE gel with Coomassie staining.

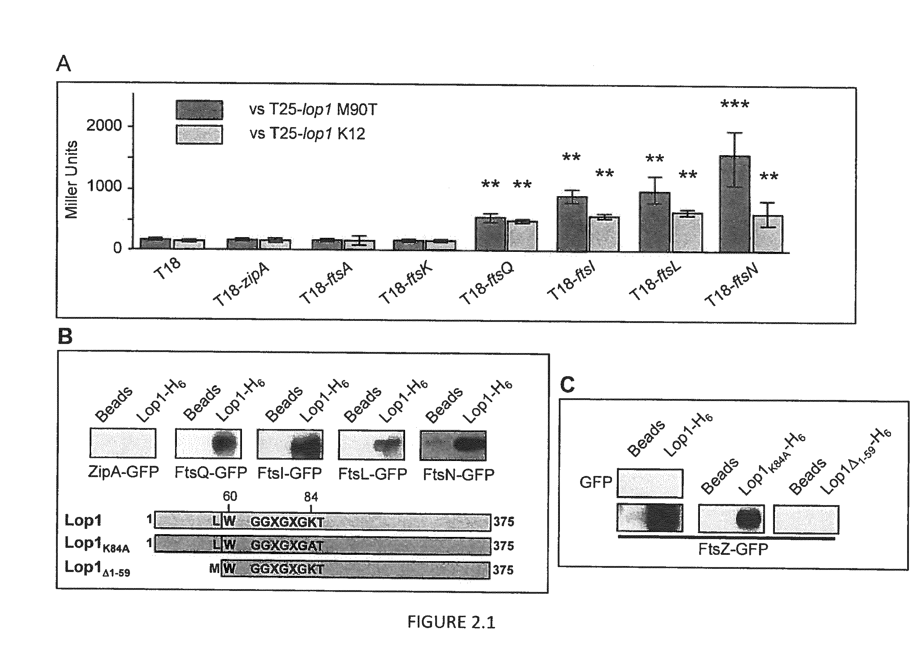

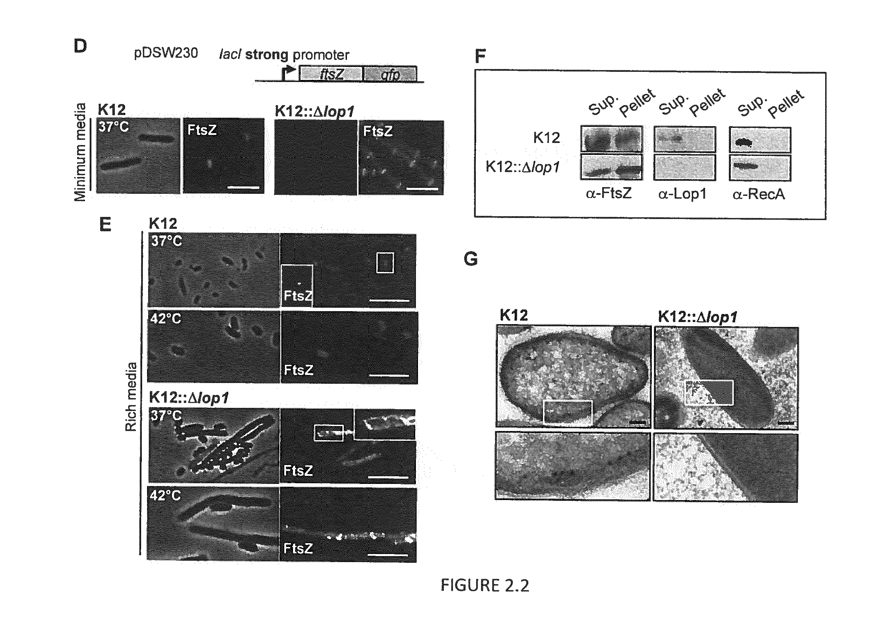

**Figure 2****. Lop1 is a cytoplasmic protein, which interacts with FtsZ and is required for Z-ring shape stabilization.**
   **(A)** A bacterial two-hybrid assays were performed using the T25-*lop1* K12 (*E. coli* K12) or T25-*lop1* M90T (*Shigella* M90T) versus T18-*zipA*/*ftsA*/*ftsK*/*ftsQ*/*ftsL*/*ftsI*/*ftsN* plasmid constructs. Results are expressed in Miller Units and averaged from three independent experiments. Error bars show the S.D.. Comparing average activity to the T18 negative control, ** indicates *p* < 0.01 and *** indicates *p* < 0.001 (Student's T-test)
   **(B)** The interaction between *E*. *coli* FtsZ-GFP and *E. coli* Lop1-H₆, Lop1_{K84A}-H₆ and Lop1Δ₁₋₅₉-H₆ respectively was analysed using an His-pullown assay. Schematic representation of Lop1, Lop1_{K84A} and Lop1Δ₁₋₅₉ key amino acids involved in ATP binding site and cleavage site.
   **(C)** The interaction between K12 Lop1-H₆ and ZipA-GFP (pDSW242), FtsQ-GFP (pDSW240), FtsI-GFP (pDSW234), FtsL-GFP (pDSW326) and FtsN-GFP (pDSW238) was analysed using an His-pulldown approach.
   **(D)** Localization of the FtsZ-GFP (pDSW230, represented in the upper panel) protein fusion in K12 wild-type (wt) and K12*::*Δ*lop1* strains grown in minimum media at 37°C in the absence of IPTG until an OD₆₀₀=0.5 was reached. Results are representative of three independent experiments. Bars are 2 µm.
   **(E)** Localization of the FtsZ-GFP in K12 and K12::Δ*lop1* strains grown in rich media (LB) at 37°C, until an OD₆₀₀=0.5 was reached. Results are representative of three independent experiments. Bars are 5 µm.
   **(F)** Western blotting of FtsZ and Lop1 in K12 and K12::Δ*lop1* strains using rabbit polyclonal antibodies on supernatant (Sup.) and pellet fractions.
   **(G)** Lop1 localization was performed in K12 and K12::Δ*lop1* strains by electron microscopy using immunogold staining with a polyclonal α-Lop1 antibody (1:1000) and Protein A gold labelled on cryosections. Bars are 200 nm.

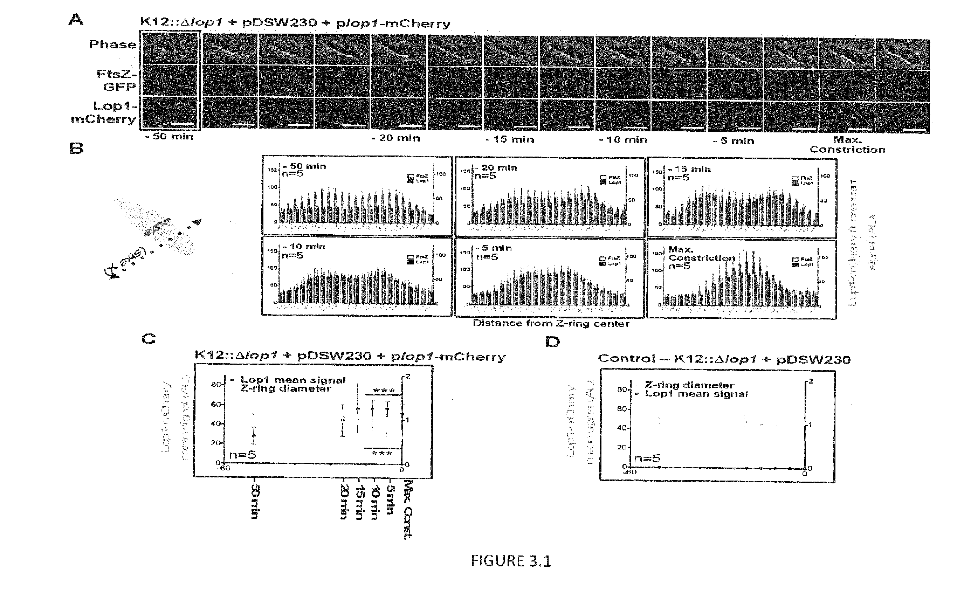

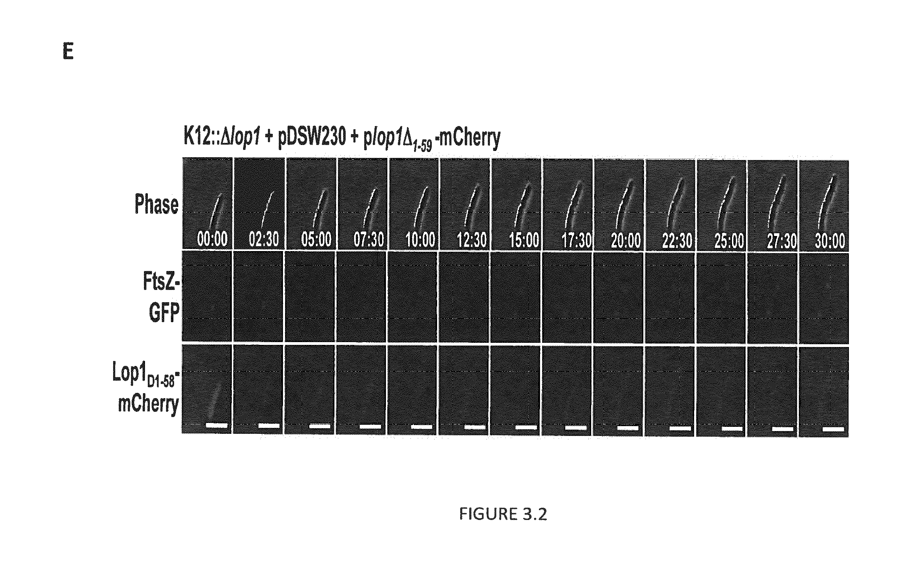

**Figure 3****. Lop1 co-localises with constricting Z-ring. The N-terminal fragment of Lop1 is required for the Z-ring association.**
   **(A)** FtsZ-GFP and Lop1-mCherry time-dependent expression and localization during a cell division process observed in a K12::Δ*lop1*/pDSW230/p*lop1-*mCherry strain. Time-lapse observation was performed on a LB-agar pad at 30°C, using a 200M Axiovert epifuorescent microscope (Zeiss). Image acquisition was performed every 3 min. This result is representative of five individual observations from three independent experiments. Bars are 2 µm.
   **(B)** FtsZ-GFP and Lop1-mCherry fluorescent signals (AU) were quantified in relation with the distance from the Z-ring center (as indicated on the lefthand scheme). Measurements were performed on images acquired at the maximal constriction (Max. constriction) and respectively 50 min, 20 min, 15 min, 10 min, 5 min before. n=5 independent observations, error bars show the S.D.
   **(C)** Lop1-mCherry mean signal (AU) and Z-ring diameter (µm) were calculated for each time-point described in (B). n=5 independent observations, error bars show the S.D.
   **(D)** Representation of the Lop1-mCherry mean signal (AU) in relation with the Z-ring diameter represented in (C). n=5 independent observations, error bars show the S.D. *** indicates statistical significance p< 0.001, (Student's T test).
   **(E)** FtsZ-GFP and Lop1Δ₁₋₅₉-mCherry time-dependent expression and localization during a cell division process observed in a K12::Δ*lop*1/pDSW230/p*lop1Δ*₁₋₅₉-mCherry strain. Time-lapse observation was performed as described in (A). This result is representative of three individual observations from three independent experiments. Timing is indicated in min. Bars are 2 µm.

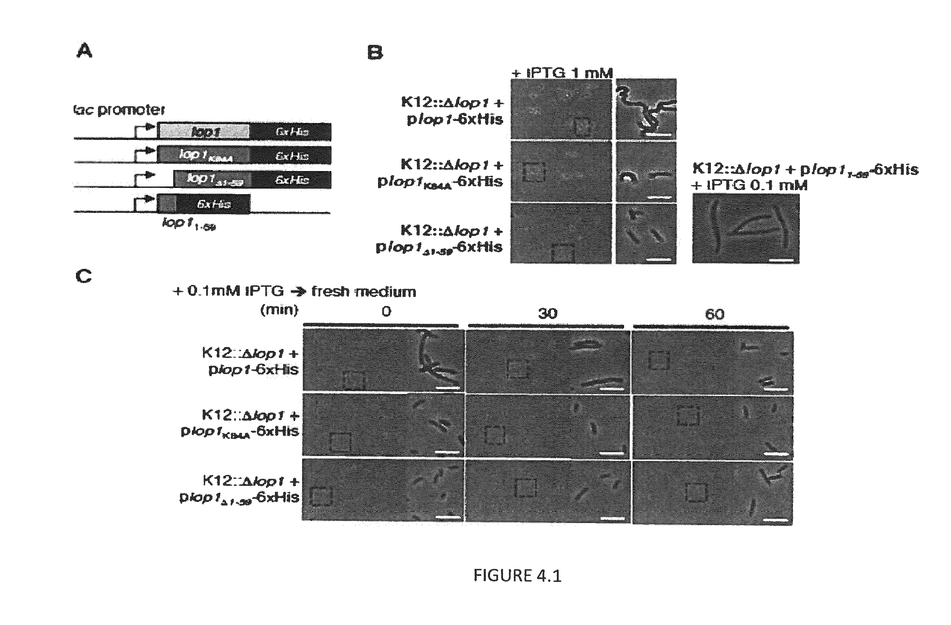

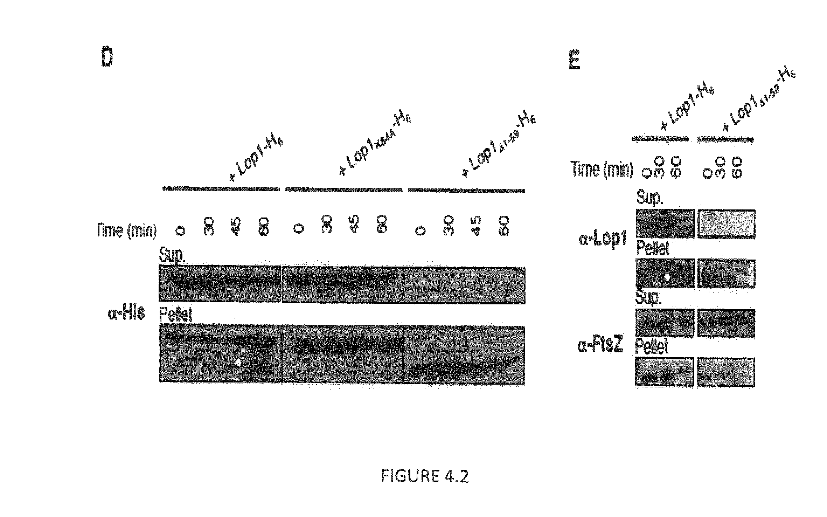

**Figure 4****. Lop1 overexpression leads to cell-shape modification. The ATP binding-site and the N-terminal 1-59 aa fragment are required for Lop1 function**
   **(A)** Schematic representation of p*lop1-*H₆*,* p*lop1_{K84A}-*H*₆,* p*lop1Δ₁₋₅₉*-H₆ and p*lop1*₁₋₅₉-H₆.
   **(B)** Cell-shape modifications of K12::Δ*lop1*/p*lop1*-H₆, K12*::Δlop1*/*plop1_{K84A}-*H₆, K12::Δ*lop1*/p*lop1Δ*₁₋₅₉-H₆ and K12::Δ*lop1*/p*lop1*₁₋₅₉-H₆ strains grown in LB liquid media at 37°C in the presence of IPTG, as indicated until the OD₆₀₀=0.5 was reached. These results are representative of at least three three independent experiments. Bars are 2 µm.
   **(C)** In order to analyse the turn-over of Lop1-H₆, Lop1_{K84A}-H₆ and Lop1Δ₁₋₅₉-H₆ proteins fusions overexpression, the constructs described in (B) were grown in LB liquid media at 37°C in the presence of IPTG 0.1M until the OD₆₀₀=0.5 was reached before growing them in a fresh LB media without IPTG (time t=0). Bars are 2 µm.
   **(D)** Western blotting of Lop1-H₆, Lop1_{K84A}-H₆ and Lop1Δ₁₋₅₉-H₆ (α-His) performed on samples described in (C) in supernatant (Sup.) and pellet fractions at time 0, 30, 45 and 60 min. These results are representative of three independent experiments. Lop1Δ₁₋₅₉ is indicated with a white arrow.
   **(E)** Western blot analysis (α-Lop1 and α-FtsZ) performed on supernatant (Sup.) and pellet fractions from K12::*Δlop1*/p*lop1-*H₆*,* K12::*Δlop1*/p*lop1_{K84A}-*H₆*,* K12::Δ*lop1*/p*lop1*Δ₁₋₅₉-H₆ described in (C) at time 0, 30 and 60 min. These results are representative of three independent experiments. Lop1Δ₁₋₅₉ is indicated with a white arrow.

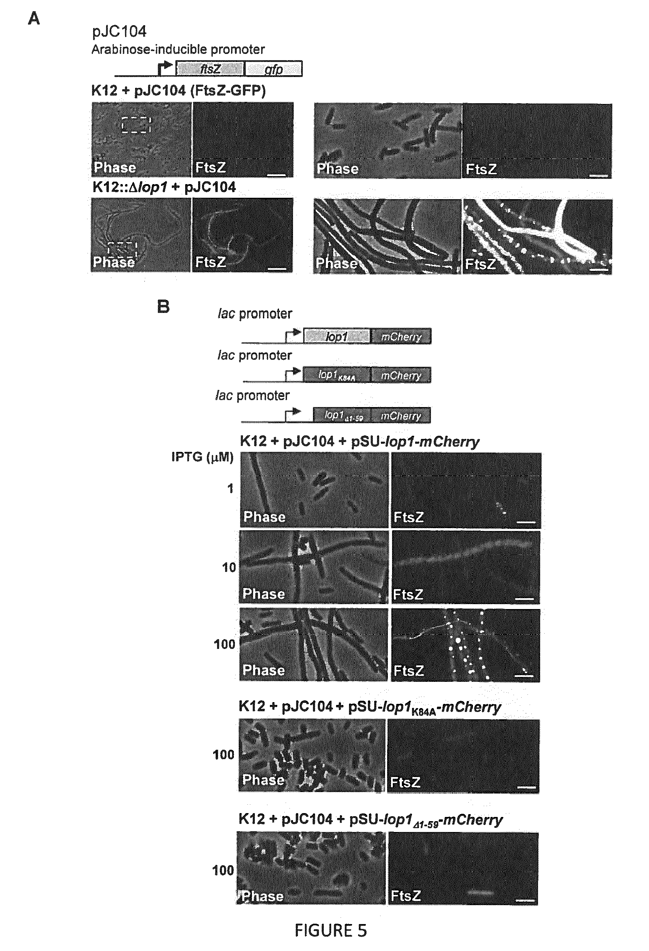

**Figure 5****. Lop1 overexpression leads to the Z-ring destabilisation.**
   **(A)** FtsZ-GFP expression using the pJC104 vector (Mukherjee et al., 2001) in the K12 and K12::Δ*lop1* strains grown in LB liquid media at 37°C in the absence of arabinose, until the OD₆₀₀=0.5 was reached. Bars are 2 µm
   **(B)** FtsZ-GFP expression (pJC104) and localization upon Lop1-mCherry (pSU-*lop1*-mCherry) and mutated forms (pSU-*lop1*_{K84A}-mCherry, pSU-*lop1Δ*₁₋₅₉-mCherry) overexpression. Bacteria were grown in LB media at 37°C in the presence of IPTG, as indicated until an OD₆₀₀=0.5 was reached. These results are representative of two independent experiments. Bars are 2 µm.

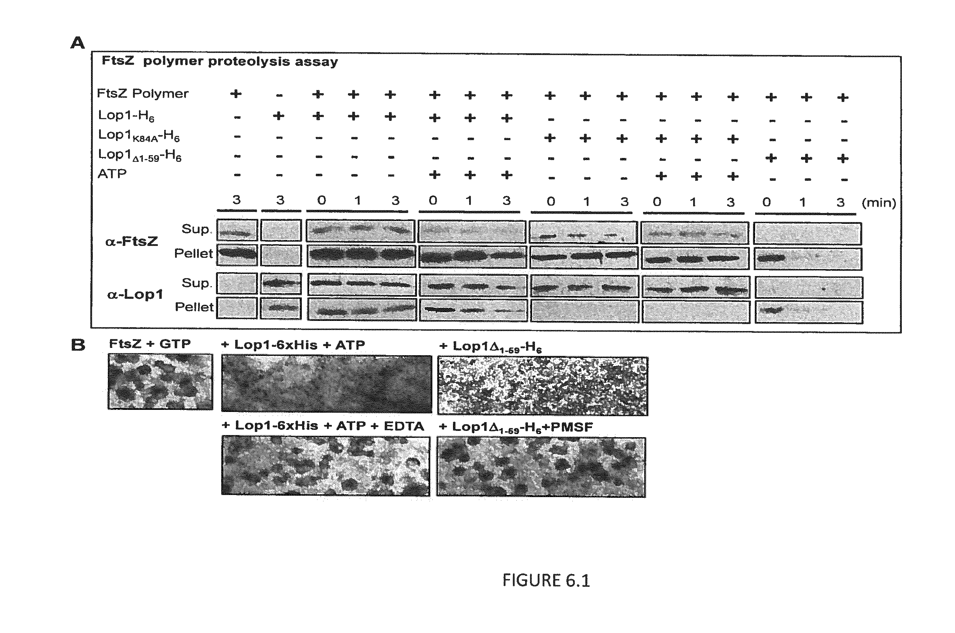

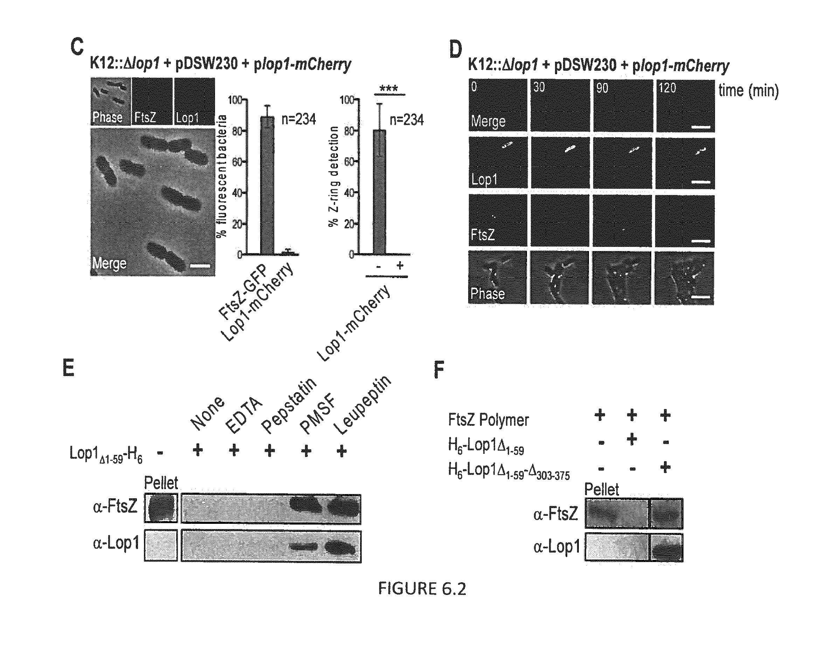

**Figure 6****. *In vitro,* Lop1Δ₁₋₅₉ catalyses the FtsZ proteolysis in an ATP-independent manner.**
   **(A)** The stability of FtsZ polymer (produced as described in Figure 16) was assessed in a reaction mixture containing 50 mM Hepes, 50 mM KCl, 5 mM MgCl₂ and equimolar quantities of Lop1-H₆, Lop1_{K84A}-H₆ or Lop1Δ₁₋₅₉-H₆ and 1 mM ATP when indicated, for 3 min at 30°C. FtsZ polymers containing pellet fraction (P) was separated from the soluble fraction (S) by ultracentrifugation. Lop1-H₆ (and mutated forms) and FtsZ were detected in both fractions by western blot using rabbit polyclonal antibodies. The results are representative of four independent experiments.
   **(B)** TEM observation of Z-ring formation was performed by negative staining in a reaction mixture containing 50 mM Hepes, 50 mM KCl, 5 mM MgCl₂ in the presence of 10 mM of CaCl₂, as indicated previously (Yu and Margolin, 1997b). The reaction occurred at 30°C during 3 min, in the presence of 1 mM GTP, 30 µg/mL purified FtsZ, and equimolar quantities of purified Lop1-H₆, Lop1_{K84A}-H₆ or Lop1Δ₁₋₅₉-H₆ and 1 mM ATP or 5 mM EDTA or 1 mM PMSF when indicated. The results are representative of three independent experiments.
   **(C)** Co-expression of FtsZ-GFP and Lop1-mCherry in the K12::Δ*lop1*/pDSW230/p*lop1*-mCherry strain grown in LB liquid media at 37°C in the absence of IPTG, until the OD₆₀₀=0.5 was reached. Bars are 2 µm. This result is representative of ten individual observations from four independent experiments. Bar is 2 µm. In graph, n represents the total number of measured bacteria. Error bars show the S.D., *** indicates *p* < 0.001 (Student's T-test)
   **(D)** K12::Δ*lop1*/pDSW230/p*lop1*-mCherry strain was grown on a LB-agar pad at 30°C in the absence of IPTG. Imaging was performed from 0 to 120 min, as indicated, using a 200M Axiovert epifuorescent microscope (Zeiss). Bars are 3 µm.
   **(E)** FtsZ polymer proteolysis by Lop1Δ₁₋₅₉-H₆ was assessed as described in (A) in the presence of 1 mM ATP and 5 mM EDTA, 2.5 µg/mL pepstatin, 1 mM PMSF or 10 µg/mL leupeptin. Lop1Δ₁₋₅₉-H₆ and FtsZ were detected in the pellet fraction by western blot using rabbit polyclonal antibodies. The results are representative of four independent experiments.
   **(F)** FtsZ polymer proteolysis by H₆-Lop1Δ₁₋₅₉ was assessed during 1 min at 30°C as described in (A) in the presence of H₆-Lop1Δ₁₋₅₉ or H₆-Lop1Δ₁₋₅₉Δ₃₀₃₃₇₅. Proteins were detected in the pellet fraction by western blot using rabbit polyclonal antibodies. The results are representative of three independent experiments.

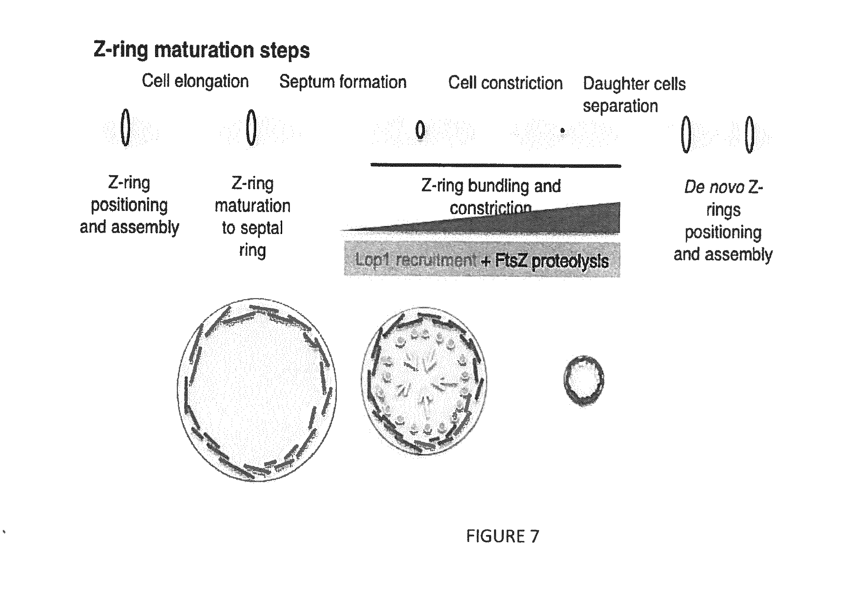

**Figure 7****. Graphical abstract. Schematic representation of the proposed model for Lopl-promoted Z-ring proteolysis, leading to its constriction.**
   In this study, the inventors demonstrate that Z-ring dynamic constriction is promoted by a novel ATPase named Loopin 1 (Lop1), according to its function. The N-terminal extremity of Lop1 is required for its interaction with FtsZ *in vitro* and in the bacteria. In an ATP-dependent manner, this N-terminal (1-59) fragment is cleaved by autoproteolysis. Lop1Δ₁₋₅₉ is a serine protease catalyzing the proteolytic cleavage of FtsZ-polymers. Taken together, these results suggest that the Z-ring constriction process is the consequence of an active proteolysis promoted by Lop1Δ₁₋₅₉ on the Z-ring. The autoproteolyis activity of Lop1Δ₁₋₅₉ observed *in vitro* is a first element of an auto-regulation of this process, allowing a new cycle of division to be initiated.
**Figure 8****. The *Shigella flexneri* 5A M90TΔ*lop1* mutant is attenuated *in vivo.***
   **(A)** Sequence comparison between the *Shigella flexneri* and *E. coli* Lop1 protein sequences (SFV3259 and B3232 (YhcM) respectively) using the ClustalW software. The GGXGVXKT ATP-binding site is highlighted in purple.
   **(B)** Competitive index (C.I.) of *Shigella flexneri* 5A *lop1* tansposon mutant (M90Tmut6), *lop1* mutant (M90T::Δ*lop1*) and complemented strain (M90T*::*Δ*lop1*/*plop1-*GFP M90T) *in vivo.* The C.I. assessed the ability of each mutant to colonize the rabbit ileal loop in comparison with the wild-type strain. A C.I. of 1 indicates no attenuation. The results are an average of at least three independent experiments.
   **(C)** Histo-pathological analysis of rabbit ileal loops infected by M90T, M90T::*Δlop1* and M90T VP- (BS176). Paraffin embedded tissues were stained using haematoxylin-eosin. Bars are 50 µm.
   **(D)** Immunodetection of the M90T and M90T::Δ*lop1* strains in the rabbit ileal loop model. DNA is stained with Dapi (blue), actin with RRX-Phalloidin (Red). *Shigella* strains are labelled using a rabbit polyclonal α-LPS antibody (green). Image acquisition was performed using a confocal microscope. Bars are 5 µm.

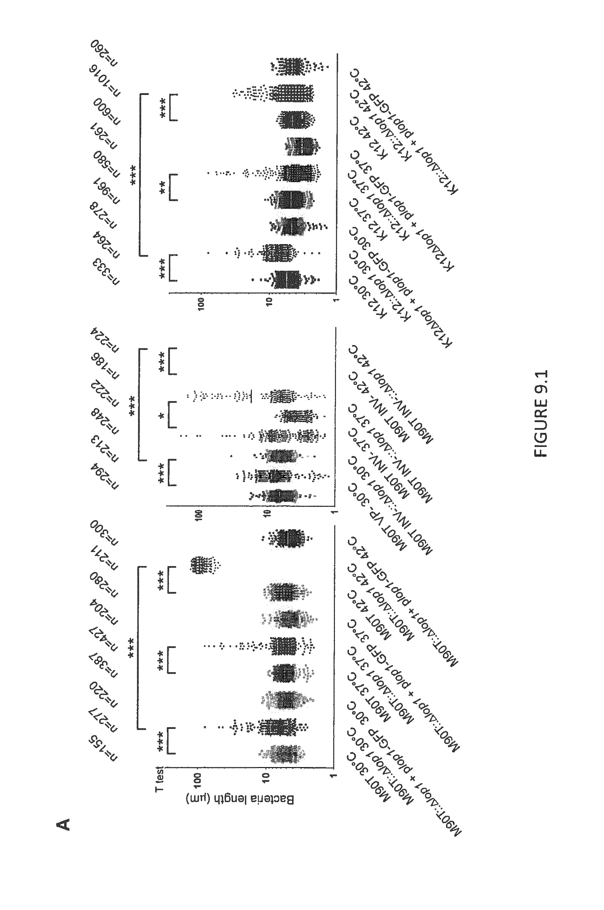

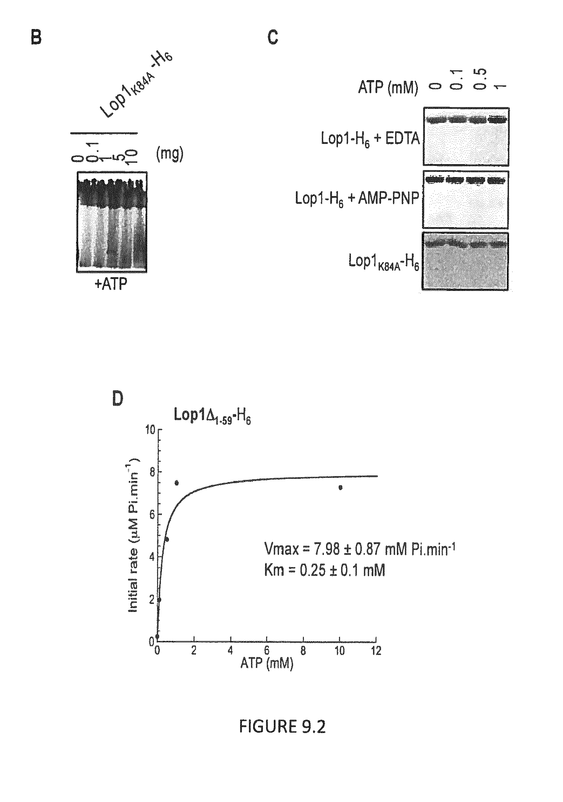

**Figure 9****. *E. coli* and *Shigella* Lop1 sequence alignment. Biochemical properties of *E*. *coli* Lop1 and Lop1Δ₁₋₅₉.**
   **(A)** Bacteria length measurement performed on each strains and conditions described in Figures 1B and 1C panels using the MicrobeTracker software (Sliusarenko et al., 2011). Three independent bacterial cultures were performed for each strain in each growth condition. n indicates the total number of measured bacteria per condition. *** indicates statistical significance < 0.001, * p<0,05 and * p<0.01 respectively (Student's T test).
   **(B)** Lop1_{K84A} ATPase activity was analysed using a silica layer chromatography technique. The reaction was performed in a TrisHCl 50 mM pH7.4 buffer containing 10 mCi of radiolabeled ATPγ32 (or GTPγ32), 10 mM ATP and 2.5 mM MgCl₂ in the presence of various Lop1_{K84A}-H₆ quantities, as indicated. The reaction occurred during 10 min at 30°C. This result is representative of three independent experiments.
   **(C)** Protein stability assay. Lop1-H₆ or Lop1_{K84A}-H₆ (80µg) were incubated at 37°C during 10 min in a TrisHCl 50 mM pH=7.4 buffer containing 5mM MgCl₂ in the presence of indicated concentration of ATP and when indicated in the presence of 5mM EDTA, 1 mM AMP-PNP. SDS-PAGE gel with Coomassie staining.
   **(D)** Enzymatic parameters (Vmax, Km) of Lop1Δ₁₋₅₉-H₆ calculation using 5µM of purified enzyme in the presence of various ATP concentrations (0.1, 0.5, 1 and 10 mM). The initial rates (µM Pi.min⁻¹) were averaged from three independent experiment performed in duplicate.

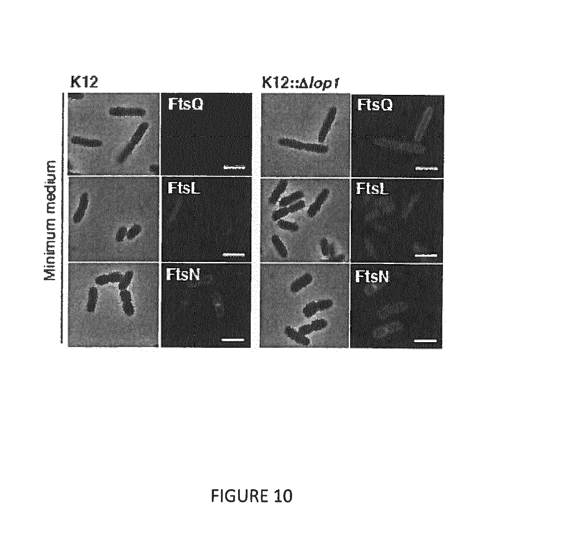

**Figure 10****. FtsQ-GFP, FtsL-GFP and FtsN-GFP localization in *E. coli* K12 wild-type and Δ*lop1* strains.**
   **(A)** Localization of FtsQ-GFP (pDSW240), FtsL-GFP (pDSW326) and FtsN-GFP (pDSW238) protein fusions in K12 wild-type (wt) and Δ*lop1* strains grown in minimum media at 37°C in the absence of IPTG (except FtsL-GFP expression with 10 µM IPTG), until an OD₆₀₀=0.5 was reached. Results are representative of three independent experiments. Bacteria were observed using a Nikon Eclipse 80i epifluorescent microscope. Bars are 2 µm.

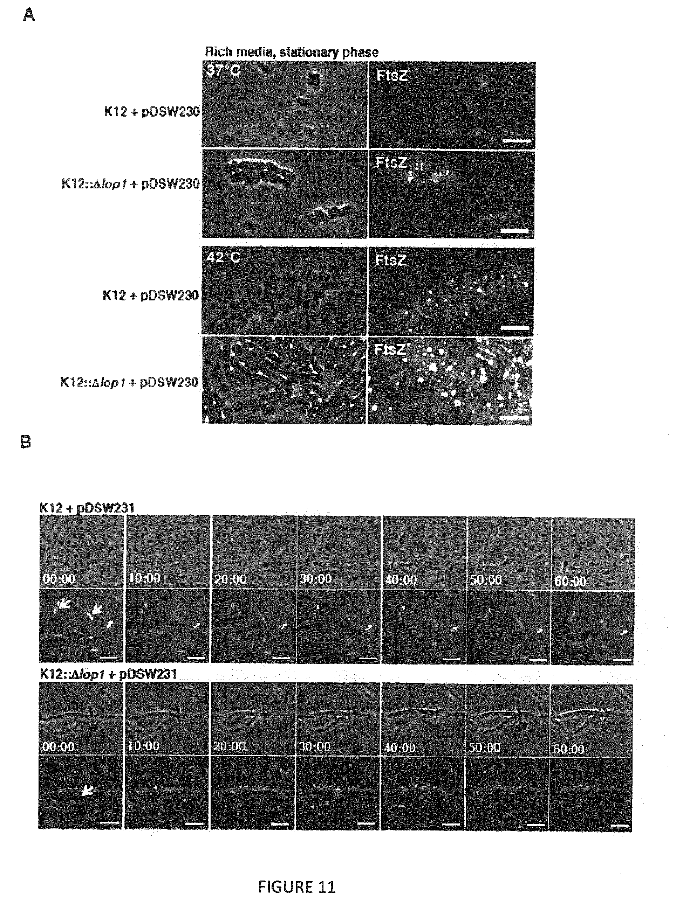

**Figure 11****. FtsZ-GFP expression in K12 and K12::Δ*lop1* strains.**
   **(A)** FtsZ-GFP (pDSW230) localization in K12 and K12::Δ*lop1* strains during stationary phase performed in LB rich media at 37°C or 42°C. These observations are representative of at least three independent experiments. Bars are 2 µm.
   **(B)** FtsZ-GFP (pDSW231) time-dependent expression and localization in *E coli* K12 wild-type and Δ*lop1* strains during a cell division process. White arrows indicate normal Z-rings. Time-lapse observation was performed on strains grown on a LB-agar pad at 30°C in the absence of IPTG, using a 200M Axiovert epifuorescent microscope (Zeiss). Image acquisition was performed every 3 min. These results are representative of at least three independent observations. Bars are 2 µm.
**Figure 12****. pSUC plasmid description.**
   Schematic representation of the pSUC plasmid map in addition with its multiple cloning site sequence (HindIII and XbaI restriction sites are underligned).
**Figure 13****. Inducible expression of Lop1-mCherry, Lop1_{K84A}**-**mCherry and Lop1Δ₁₋₅₉-mCherry in *E. coli* K12.**
   Inducible overexpression of Lop1-mCherry and mutated forms in K12 (pSU*lop1*-mCherry, pSU*lop1*_{K84A}-mCherry and pSU*lop1Δ₁₋₅₉*-mCherry, representative scheme). Bacteria were grown in LB media at 37°C in the presence of IPTG, as indicated until an OD₆₀₀=0.5 was reached. The observations were performed using a Nikon Eclipse 80i epifluorescent microscope. These results are representative of two independent experiments. Bars are 2 µm.

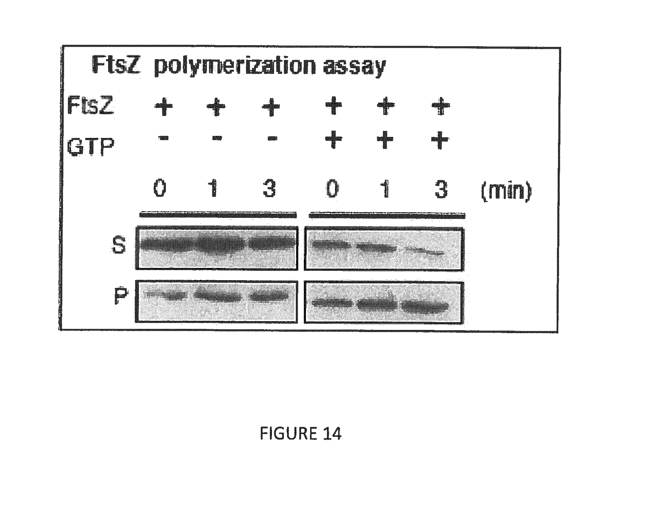

**Figure 14****. FtsZ polymerization assay.**
   FtsZ polymerization assay was performed in a reaction mixture containing 50 mM Hepes, 50 mM KCl, 5 mM MgCl₂ in the presence of 0.5 µg/mL purified FtsZ and 1 mM GTP when indicated. The reaction was performed at 30°C during 3 min. FtsZ polymers containing pellet fraction (P) was separated from the soluble fraction (S) by ultracentrifugation. FtsZ was detected in both fractions by western blot using a rabbit polyclonal antibody. The results are representative of four independent experiments.

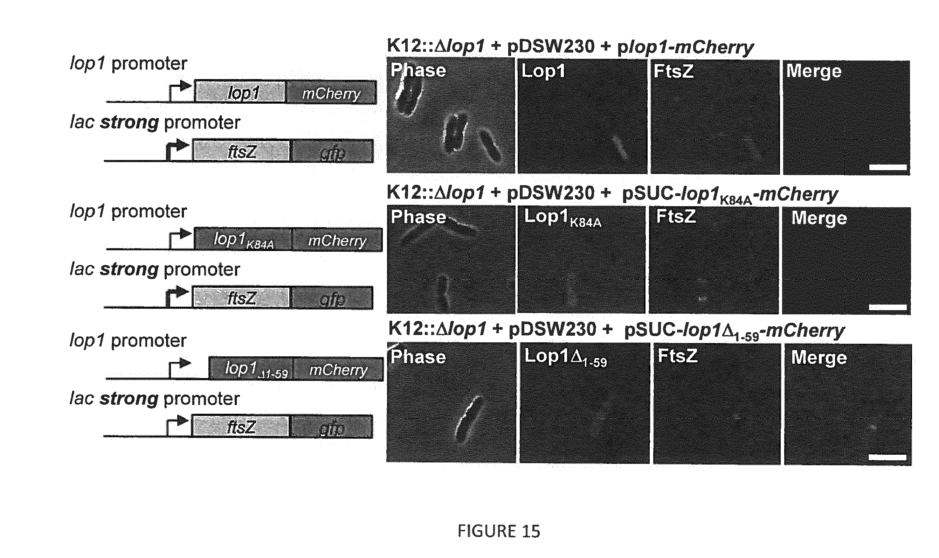

**Figure 15****. Lop1_{K84A} and Lop1Δ₁₋₅₉ expression do not prevent Z-ring formation**
   Lop1-mCherry and mutative forms expression and Z-ring formation observation in K12::Δ*lop1*/pDSW230/p*lop1*-mCherry, K12::Δ*lop1*/pDSW230/p*lop1*_{K84A}-mCherry and K12::Δ*lop1*/pDSW230/p*lop1Δ*_{*1*-59}-mCherry strains (schematic representation) grown in LB media without IPTG in a LB rich media at 37°C.
**Figure 16****. Lop1 do not interact with ClpP *in vitro.***
   Gel filtration analysis of the Lop1-H₆ and ClpP-H₆ interaction. **(A)** 2mg of each His-tagged protein was incubated in Buffer A prior gel filtration analysis. **(B)** SDS-PAGE analysis of each detected peak between 87 and 93 mL corresponding to the Lop1-H₆ K12 elution fraction and 111 and 117 mL corresponding to the ClpP-H₆ elution fraction. SDS-PAGE gel was stained with a Coomassie staining.

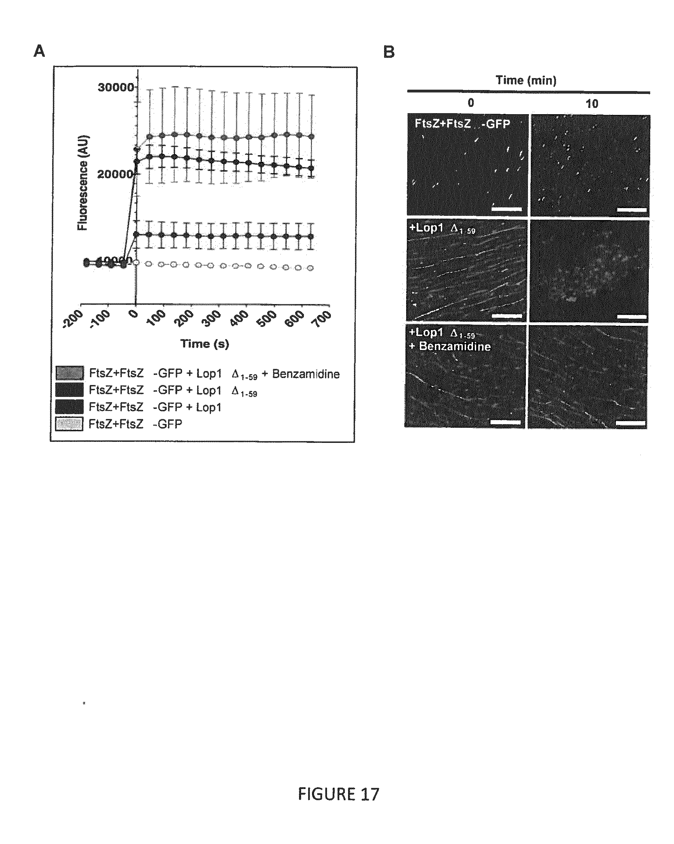

**Figure 17****: In vitro fluorescence-based assay**
   As Lop1 overexpression seemed to perturb the Z-ring constriction, we aimed at deciphering whether Lop1 and Lop1Δ₁₋₅₉ act directly on FtsZ polymers *in vitro.* We designed a fluorescence-based assay as polymerization of a FtsZ/FtsZ-GFP mixture leads to an increase of the solution fluorescence (Trusca and Bramhill, 2002). In the presence of GTP, purified FtsZ and FtsZ-GFP form polymers, as described previously (Yu and Margolin, 1997b); however the level of the detected fluorescence remains low and the polymers length was reduced (Figures 17A and 17B). Indeed upon equimolar addition of Lop1 or Lop1Δ₁₋₅₉ we could observe a significant increase of the solution fluorescence (Figure 17A). Full-length Lop1 addition promoted FtsZ/FtsZ-GFP bundling and the formation of large helical three-dimensional polymerized structures (Figure 17B t=0). These structures remained stable over time in the absence of ATP (t=10min), while they remain no longer stable in the presence of ATP (Figure 17B, t=10min). This observation was correlated with a decrease of the solution fluorescence in in the fluorescence-based assay (Figure 17A, Student's T test p<0.01), which was consistent with the ATP-dependent autoproteolytic maturation of Lop1 described previously (Figure 1E). Alternatively, in the presence of Lop1Δ₁₋₅₉, while we similarly observed a rapid and significant increase of the solution fluorescence level (Figure 17A), we could not observe the FtsZ/FtsZ-GFP helical structures formation, however we could visualize the formation of a homogeneous and dense FtsZ/FtsZ-GFP polymer network (Figure 17B, t=0). Interestingly, these polymers were rapidly degraded (Figure 17B, t=10min), which was correlated with a significant decrease of the fluorescence level (Figure 17C, Student's T test p<0.01). As a control, the simultaneous addition of benzamidin with Lop1Δ₁₋₅₉ did not impair the fluorescence increase associated to the formation of the FtsZ/FtsZ-GFP polymer network (Figure 17A and 17B), although preventing its degradation (Figure 17B), in association with a stable level of the fluorescent signal (Figure 17A). This experiment showing an inhibition of the proteolytic activity of Lop1Δ₁₋₅₉ by benzamidin suggested that this protein has a serine protease activity.

There will now be described by way of example a specific mode contemplated by the Inventors. In the following description numerous specific details are set forth in order to provide a thorough understanding. It will be apparent however, to one skilled in the art, that the present invention may be practiced without limitation to these specific details. In other instances, well known methods and structures have not been described so as not to unnecessarily obscure the description.

### Example 1 - EXPERIMENTAL PROCEDURES

### Expression plasmid construction

The pSUC vector construction was made by amplifying the mCherry fusion from the pmCherry-N1 vector using the SG150 (SEQ ID NO: 11) and SG151 (SEQ ID NO: 12) primer pair (Table 2), introducing the BamHI and EcoRI restriction sites. The pSU19 vector was digested with BamHI and EcoRI restriction enzymes prior ligation of the digested mCherry amplified fragment, leading to the generation of the pSUC vector. This expression vector allows the expression of mCherry protein fusions in C-terminal under the control of the gene of interest promoter in *E*. *coli* and in *Shigella.*

This plasmid allowed the expression of Lop1-mCherry fusion under the control of the *lop1* promoter (see below).

The expression of the FtsZ-GFP fusion under the control of a *lacI* promoter was performed using either the pDSW230 and pDSW231 constructs or pJC104 (Mukherjee et al., 2001) (kindly provided by Pr. Lutkenhaus) (described in Table 1).

**Table 1.**

| **Plasmids and strains** | **Description** | **Source/Ref.** |
|---|---|---|
| pUT18 | Two hybrids expression vector (Amp^{r}) | (Karimova et al., 1998) |
| pKT25 | Two hybrids expression vector (Km^{r}) | (Karimova et al., 1998) |
| pKD46 | Red recombinase expression plasmid | (Datsenko and Wanner, 2000) |
| pSU19 | Expression vector, lacI inducible promoter (Cm^{r}) | (Martinez et al., 1988) |
| pmCherry-N1 | Expression vector, Cterm mCherry fusion | Addgene |
| pSUC | Expression vector, Cterm mCherry fusion (Cm^{r}) | This study |
| pKJ1 | Expression vector (Amp^{r}) | Addgene |
| pFpV25 | Expression vector, Cterm GFP fusion (Amp^{r}) | (Valdivia and Falkow, 1996) |
| pNIC28-Bsa4 | Expression vector, (Km^{r}), N-Terminal 6xHis (H₆) tag fusion, TEV protease cleavage site | |
| M90T | *S. flexneri* (serotype 5a), nalidixic acid resistant | (Sansonetti et al., 1982) |
| M90T INV- | *S. flexneri* (serotype 5a) avirulent strain (BS176) | (Sansonetti et al., 1982) |
| M90T *mut6* | | (West et al., 2005) |
| *E*. *coli* K12 | *E. coli* K12 MG 1655 | |
| M90T::Δ*lop1* | *S. flexneri* 5A M90T *lop1* mutant | This study |
| *E. coli* K12*::*Δ*lop1* | *E. coli* K12 MG 1655 *lop1* mutant | This study |
| M90T INV-::Δ*lop1* | *BS176*Δ*lop1; S. flexneri* 5A *without virulence plasmid (VP-) lop1* mutant | This study |
| p*lop1*-GFP M90T | pFpV25-*lop1* M90T | This study |
| p*lop1*-GFP K12 | pFpV25-*lop1* K12 | This study |
| p18*-zipA* | pUT18-*zipA* K12 | (Karimova et al., 2005) |
| p18-*ftsA* | pUT18-*ftsA* K12 | (Karimova et al., 2005) |
| p18-*ftsK* | pUT18-*ftsK* K12 | (Karimova et al., 2005) |
| p18-*ftsQ* | pUT18-*ftsQ* K12 | (Karimova et al., 2005) |
| p18*-ftsL* | pUT18-ftsL K12 | (Karimova et al., 2005) |
| p18*-ftsI* | pUT18-ftsI K12 | (Karimova et al., 2005) |
| p18-*ftsN* | pUT18-ftsN K12 | (Karimova et al., 2005) |
| p25-*lop1* M90T | pKT25-*lop1* M90T | This study |
| p25*-lop1* K12 | pKT25-*lop1* K12 | This study |
| pDSW231 | FtsZ-GFP inducible expression (weak strength promoter) | (Weiss et al., 1999) |
| pDSW230 | FtsZ-GFP inducible expression (high strength promoter) | (Weiss et al., 1999) |
| pDSW242 | ZipA-GFP inducible expression (high strength promoter) | (Chen et al., 1999) |
| pDSW240 | FtsQ-GFP inducible expression (high strength promoter) | (Chen et al., 1999) |
| pDSW236 | FtsL-GFP inducible expression (high strength promoter) | (Karimova et al., 1998; Ghigo et al., 1999) |
| pDSW234 | Ftsl-GFP inducible expression (high strength promoter) | (Karimova et al., 1998; Weiss et al., 1999) |
| pDSW238 | FtsN-GFP inducible expression (high strength promoter) | D. Weiss collection |
| p*lop1*-H₆ | pKJ1-*lop1*. Overexpression of *E*. *coli* K12 Lop1-H₆ | This study |
| p*lop1*_{K84A} -H₆ | pKJ1-*lop1*_{K84A}. Overexpression of *E*. *coli* K12 Lop1 _{K84A}-H₆ | This study |
| p*lop1Δ₁₋₅₉-*H*₆* | pKJ1*-lop1*Δ*₁₋₅₉.* Overexpression of *E*. *coli* K12 Lop1*Δ*₁₅₉-H₆ | This study |
| p*lopl₁₋₅₉-H₆* | pKJ1*-lop1₁₋₅₉.* Overexpression of *E. coli* K12 Lop1 ₁₋₅₉-H₆ (N-terminal fragment) | This study |
| p*lop1*-mCherry | pSUC*-lop1.* Expression of *E*. *coli* Lop1-mCherry fusion under *lop1* promoter control (-300bp) | This study |
| p*lop1*_{K84A}-mCherry | pSUC-*lop1*_{*K*84A}. Expression of *E. coli* Lop1_{K84A}-mCherry fusion under *lop1* promoter control (-300bp) | This study |
| p*lop1*Δ_{*1-*59}-mCherry | pSUC*-lop1Δ₁₋₅₉.* Expression of *E*. *coli* K12 Lop1Δ₁₋₅₉-mCherry fusion under *lop1* promoter control (-300bp) | This study |
| pSU*-lop1-mCherry* | pSU19*-lop1-mCherry.* Expression of *E. coli* Lop1-mCherry fusion under a *lac* promoter control | This study |
| pSU-*lop1*_{K84A}*-mCherry* | pSU19*-lop1_{K84A}-mCherry.* Expression of *E*. *coli* Lop1_{K84A}-mCherry fusion under a *lac* promoter control | This study |
| pSU*-lop1Δ₁₋₅₉-mCherry* | pSU19-*lop1Δ₁₋₅₉-mCherry.* Expression of *E. coli lop1*Δ*₁₋₅₉*-mCherry fusion under a *lac* promoter control | This study |
| pJC104 | Expression of *ftsZ-gfp,* arabinose inducible promoter | (Datsenko and Wanner, 2000; Mukherjee et al., 2001) |
| pET11a-ftsZ | Expression of FtsZ, lac promoter, AmpR | (Yu et al., 1997) |
| pNIC28-Bsa4-*lop1Δ*₁₋₅₉ | Overexpression of K12 H₆-Lop1*Δ₁₋₅₉* | This study |
| pNIC28-Bsa4-*lop1 Δ₁₋₅₉Δ₃₀₃375* | Overexpression of K12 H₆*-*Lop1*Δ₁₋₅₉Δ₃₀₃₋₃₇₅* | This study |
| pNB140 | Expression of ClpP-H₆ (pET28-*clpP*) | (Benaroudj et al., 2011) |

**Table 2**

| **Name** | **Sequence** | **Purpose** | **SEQ ID NO** |
|---|---|---|---|
| NG1281 | CAAGGAATAACAATACTGCAGGGCAAAGCGTTAC CCCAACATCG | cloning *Shigella* and *E*. *coli lop1* in pKT25 | 1 |
| NG1282 | TTGTGATTTGTGGGGATCCTTAACCCGCCAAATGC TCGCGC | cloning *Shigella* and *E*. *coli lop1* in pKT25 | 2 |
| SG127 | GCAACGCCGGATCCTGGCGTAGTTTACGATTACCA | cloning *Shigella* and *E*. *coli lop1* in pFpV25 | 3 |
| SG128 | GTGATTTGTGGCAGCATATGACCCGCCAAATGCTC GC | cloning *Shigella* and *E*. *coli lop1* in pFpV25 | 4 |
| SG114 | GTGGGGCGGTGTAGGACGCGGGCAACCTGGCTG ATGGACC | point mutation K84A in LOP1 | 5 |
| SG115 | GGTCCATCAGCCAGGTTGCCCCGCGTCCTACACCG CCCCAC | point mutation K84A in LOP1 | 6 |
| SG90 | TTCAAGGAATAACAATAAGACCATGGAAAGCGTT ACCCCA | cloning *lop1* in pKJ1 | 7 |
| SG91 | GTGATTTGTGGCAGGTTGGATCCCGCCAAATGCTC GCGC | cloning *lop1* in pKJ1 | 8 |
| SG157 | GGGCTAATGGCGCGGGTCGGTACCATGGGGGGTA AACGCG | cloning *lop1Δ₁₋₅₉* in pKJ1 | 9 |
| SG169 | GGCGTATGCTTTGTGTCTTCGCGTTTGGATCCCAG CTTACCGACCCGCG | cloning *lop1₁₋₅₉* (N-terminal fragment) in pKJ1 | 10 |
| SG150 | CCCGGGATCCACCGGTCGCCACC | Amplifying mCherry from pmCherry-N1 | 11 |
| SG151 | GATTATGATCTGAATTCGCGGCCGCT | Amplifying mCherry from pmCherry-N1 | 12 |
| SG127 | GCAACGCCGGATCCTGGCGTAGTTTACGATTACCA | Cloning lop1 in pFpV25 (500 bp upstream start codon) | 3 |
| SG128 | GTGATTTGTGGCAGCATATGACCCGCCAAATGCTC GC | Cloning lop1 in pFpV25 (500 bp upstream start codon) | 4 |
| SG219 | CGCGCCAGTACGAAGCTTGCCGGATGCGCC | cloning *lop1* in pSUC (500 bp upstream start codon) | 13 |
| SG155 | GTGGCAGTTCTAGACCCGCCAAATGCTCGCGC | cloning *lop1* in pSUC (500 bp upstream start codon) | 14 |
| SG164 | TTATTCAAGGAATAACAATAAGATCATGTGGGGTA AACGCGAAGACACAAAGC | truncating *lop1* (*Δ₁₋₅₉*) | 15 |
| SG165 | GCTTTGTGTCTTCGCGTTTACCCCACATGATCTTAT TGTTATTCCTTGAATAA | truncating *lop1* (*Δ₁₋₅₉*) | 16 |
| SG278 | GGATCCATGCAAAGCGTTACCCCAACATCGCA | cloning *lop1-mCherry* and *lop1_{K84A}*-*mCherry* in pSU 19 | 17 |
| SG328 | GGATCCATGTGGGGTAAACGCGAAGACACAAAGC | cloning *lop1*Δ₁₋₅₉-mCherry in pSU19 | 18 |
| SG329 | GAATTCTTAGCTACTTGTACAGCTCGTCCATGCC | cloning *lop1-mCherry, lop1_{K84A}*-*mCherry* and *lop1Δ₁₋₅₉-mCherry* in pSU19 | 19 |
| NWpr23 | ATGTTCATGACCTGGGAATAT | Upstream primer for the amplification of *lop1* | 20 |
| NWpr24 | GTCGCGCTTCGCGCCAGTACG | Downstream primer for the amplification of *lop1* | 21 |
| NWpr40 | ACAGCGTAGTAAAAGAGACC | Upstream primer for amplify of *dgcF* with 1022 bp flanking regions | 22 |
| NWpr41 | CGGAAACAATGCCAGAGGTG | Downstream primer for amplify of *dgcF* gene with 715 bp flanking sequences | 23 |
| SG154 | TACTGCAACGCCTGAAGCTtGCGTAGTTTACGAT | | |

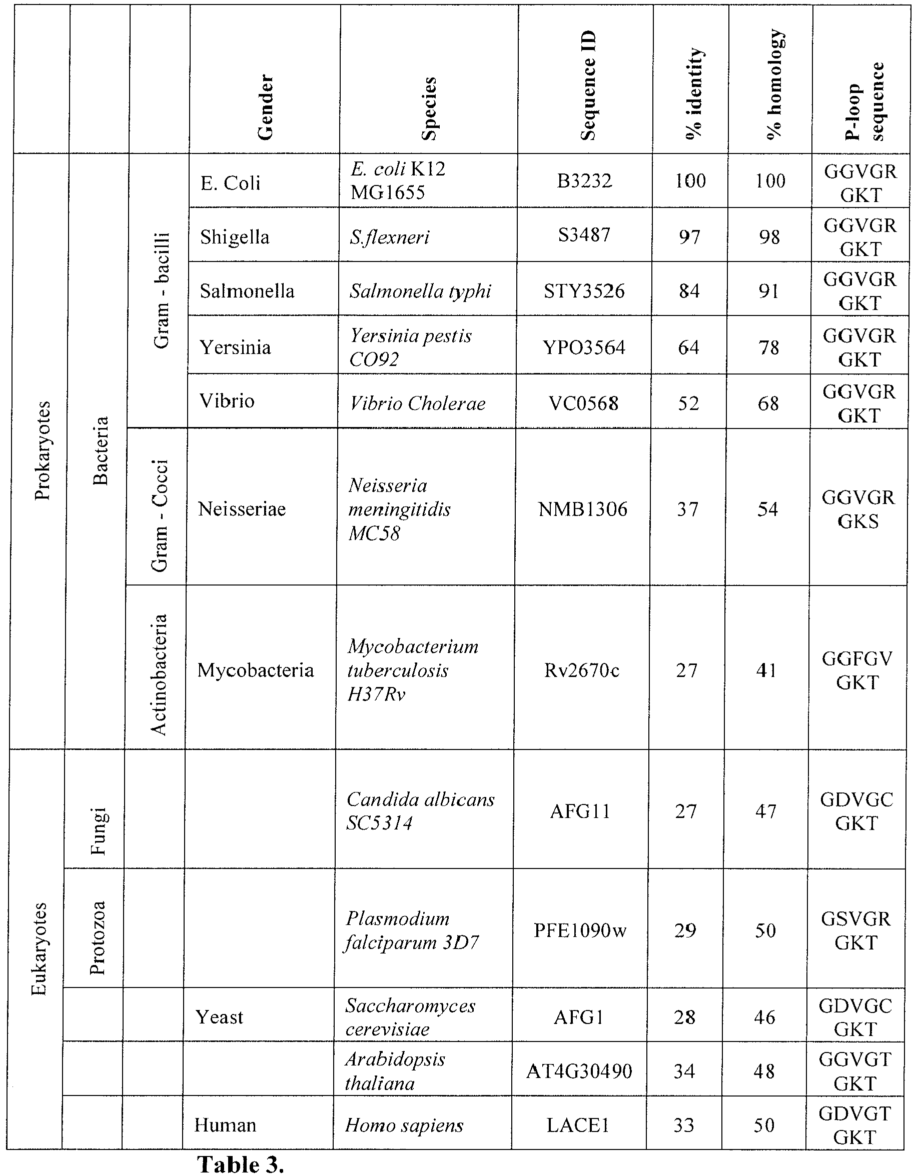

### Proteins overexpression and purification

K12 Lop1-6xHis, Lop1_{K84A}-6xHis, Lop1Δ₁₋₅₉-6xHis and ClpP-6xHis protein fusions were expressed using the p*lop1*-6xHis K12, p*lop1_{K84A}-*6xHis K12, p*lop1Δ*_{*1-5*9}-6xHis K12 and pNB140 constructs (see Table 1) expressed in an *E. coli* BL21DE3 strain. Proteins purifications are described below.

The Lop1 L₅₉/W₆₀ cleavage site identification was performed by automated N-terminal sequence analysis on a Procise ABI 470 (Applied Biosystems).

Native proteins or protein fusions were overexpressed in an *E. coli* BL21DE3 strain. Overnight cultures were subcultured in fresh LB media (1:100) and grown at 37°C until the OD₆₀₀=0.5 was reached. Overexpression was induced by the addition of 0.5 mM IPTG and was performed overnight at RT. Lop1-H₆, Lop1_{K84A}-H₆, Lop1Δ₁₋₅₉-H₆ His-Tagged proteins were purified on Talon beads (Clontech) and further purified by gel filtration using an Hiload 16/60 Superdex 200 column (GE) in a Tris 50mM pH7.5 buffer containing 5 mM MgCl₂, 1 mM EDTA and 0.1M NaCl. FtsZ and FtsZ-GFP were purified by ion exchange on a Hiload 16/10 DEAE column using a Tris 50mM pH7.5 buffer containing 5 mM MgCl₂, 1 mM EDTA and 0.1M NaCl (Buffer 1) and Tris 50mM pH7.5 buffer containing 5 mM MgCl₂, 1 mM EDTA and 1M KCl (Buffer2) as described previously (Yu et al, 1997), followed by a gel filtration, as described above.

### FtsZ polymers proteolysis assay

FtsZ polymers (P) were generated as described below during 3 min at 30°C and collected by ultracentrifugation (11 min, 80K) at 4°C (Beckman, TL-100 Ultracentrifuge). Then, the reactive buffer was discarded and replaced by a reaction mixture containing 50 mM Hepes, 50 mM KCl, 5 mM MgCl₂ in addition with 1 mM ATP and 0.5 µg/mL of purified Lop1-6xHis, Lop1_{K84A}-6xHis or Lop1/Δ₁₋₅₉-6xHis when indicated in a final volume of 100 µL. The reaction was stopped after 0, 1 or 3 min, as indicated. PMSF (Sigma-Aldrich), EDTA (Sigma-Aldrich), peptstatin (Sigma-Aldrich), leupeptin (Calbiochem) or PMSF (Roche) were added when indicated. FtsZ polymers containing pellet fraction (P) was separated from the soluble fraction (S) by ultracentrifugation (11 min, 80K) at 4°C (Beckman, TL-100 Ultracentrifuge). Samples were re-suspended in a Laemli buffer 1X final and subsequently subjected to SDS-PAGE gel analysis and transfer onto a nitrocellulose membrane. FtsZ and Lop1-6xHis, Lop1_{KS4A}-6xHis or Lop1Δ₁₋₅₉-6xHis were detected in both fractions by Western blot using rabbit polyclonal antibodies (see below).

Fluorescent FtsZ/FtsZ-GFP polymers were generated in a buffer containing 50 mM Hepes, 50 mM KCl, 5 mM MgCl₂, 10 mM CaCl₂ in addition with 1 mM GTP. Polymerization of FtsZ (100 µM) and FtsZ-GFP (50 µM) occurred during 3 min at 30°C in 96-well plates (Greiner Bio One).

Then, Lop1-H₆, Lop1_{K84A}-H₆ or Lop1Δ₁₋₅₉-H₆ (100 µM) was added to reach a in a final volume of 100 µL. The fluorescence was quantified over the time (10min, acquisition every 45s) using a SLM 8000C fluorimeter (SLM Instruments). The experiments were performed in triplicate on three independent occasions. As a negative control 1mg/mL benzamidine was added at the initial step, when indicated.

Additionally, a similar experiment was performed on glass slides to visualise the formation and proteolysis of FtsZ/FtsZ-GFP polymers using a TCS SP5 confocal microscope (Leica).

FtsZ/FtsZ-GFP polymers containing pellet fraction (P) was separated from the soluble fraction (S) by ultracentrifugation (11 min, 80K) at 4°C (Beckman, TL-100 Ultracentrifuge). Samples were re-suspended in a Laemli buffer 1X final and subsequently subjected to SDS-PAGE gel analysis and transfer onto a nitrocellulose membrane. FtsZ and Lop1-H₆, Lop1_{K84A}-H₆ or Lop1Δ₁₋₅₉-H₆ were detected in both fractions by Western blot using rabbit polyclonal antibodies FtsZGFP was detected using an anti-GFP antibody (Sigma-Aldrich).

### EM observation of Z-ring formation in vitro

In order to allow FtsZ to polymerize as a proper ring (Z-ring), 10 mM of CaCl₂ were added into a reaction mixture containing 50 mM Hepes, 50 mM KCl, 5 mM MgCl₂, as described previously (Yu and Margolin, 1997b; Camberg et al., 2009) (Mateos-Gil et al., 2012). The reaction occurred onto during 5 min at 30°C in the presence of 30 µg/mL of purified FtsZ and of an equimolar quantity of purified Lop1-6xHis, Lop1_{K84A}-6xHis or LoplΔ₁₋₅₉-6xHis and 1 mM ATP as indicated. As polymerized proteins might be unstable, the reaction occurred directly on glow discharged copper grids and the reaction was stopped by immersion of the grids in a 2% uranyl acetate solution (see below).

### Bacterial strains and growth conditions

The bacterial strains and plasmids used in this study are described in Table S1. *Shigella* strains (including *S. flexneri*) were grown in trypticase soy (TCS) broth or on TCS agar plates supplemented with 0.01% Congo Red (Sigma), when necessary. *E. coli* strains, as well as *Salmonella thyphi* were grown in LB media.

### DNA manipulations

The initial transposon insertion in S. *flexneri* M90T in *lop1* was performed as described previously (West et al., 2005). The construction of inactivated *lop1* mutants was then performed in *E*. *coli* and *S*. *flexneri.*

*Δlop1 mutants construction.* In MG1655 *E. coli* K12 Plvir page lysate was prepared on the donor strain JW3201 from the Keio collection (Baba *et al,* 2006) as described (Miller J.H., 1992). In JW3201 strain, the *lop1* ORF (open reading frame) is substituted by the kanamycin-resistance marker (Δ*lop1*::Kam) (Baba *et al,* 2006). The cassette Δ*lop1*::Kam was introduced into MG1655 by P1 transduction (Miller, J. H. 1992) and selection for kanamycin-resistant (Km^{r}) colonies was made on LB plates containing kanamycin (50 µg/ml). After re-isolation, several clones were verified by PCR to confirm the right chromosomal structure of the Δ*lop1*::kan deletion. One clone was chosen and named K12Δ*λop1::*Km.

K12::Δ*lop1* was then obtained from K12Δ*lop1::*Km by removing the kanamycin-resistance marker from the Δ*lop1*::Km cassette. In this Δ*lop1*::Km cassette, the antibiotic-resistance marker is flanked by two direct *frt* repeats, which are the recognition targets for the site specific recombinase FLP (Baba *et al,* 2006). Therefore, to get rid of the resistance marker from the K12::Δ*lop1*::Km chromosome, a temperature-sensitive plasmid pCP20 that encodes the FLP recombinase was used (Cherepanov & Wackernagel, 1995). Briefly, K12Δ*λop1::*Km cells were transformed with pCP20, and chloramphenicol-resistant (Cm^{r}) colonies were selected at 30°C on LB plates containing the corresponding antibiotic (30 µg/ml). Several of these clones were grown overnight on antibiotic-free LB plates at 42°C. Ten independent colonies were selected and after single-colony passage at 30°C, all ten colonies were no longer Cm^{r} and Km^{r}, indicating simultaneous loss of pCP20 and the kanamycin-resistance marker from the bacterial chromosome. This FLP-catalysed excision created an in-frame deletion of the *lop1* ORF, leaving behind a 102-bp scar sequence (Δ*lop1*::*frt*) (Baba *et al,* 2006). To confirm the correct chromosomal structure of the Δ*lop1*::*frt* deletion several Cm^{s} and Km^{s} clones were tested by PCR using the NWpr40 and NWpr41 primer pair (Table 2). After confirmation, one clone was chosen and named K12::Δ*Lop1*.

In order to inactivate *lop1* in *Shigella flexneri* (M90T), a one-step chromosomal inactivation method was used to target homologous region for integration. Therefore, the inventors generated PCR products with much longer flanking sequence using the K12::Δ*lop1* null mutant as the template. The M90T was transformed with PCR products amplified from K12::Δlop1::Km mutant genomic DNA using primers NWpr23 and NWpr24 (Table 2). The primers NWpr23 (SEQ ID NO: 20) and NWpr24 (SEQ ID NO: 21) were designed to include 50 bp upstream and downstream sequence flanking *lop1.* This product was transformed into M90T::pKD46 which resulted in all kanamycin resistant colonies containing the 1.5 kb kanamycin resistance gene when analysed by PCR. Thus, a *S*. *flexneri* null mutant was successfully generated (M90T::Δ*lop1*).

Expressing respectively a *lop1-GFP* and a *lop1-mCherry* fusion under the control of *lop1* promoter performed the complementation of the M90T::Δ*lop1* and K12::Δ*lop1* mutants. In order to express a Lop1-GFP fusion, the *lop1* gene of *Shigella* and *E*. *coli* and their promoters (≈500bp) were amplified with the SG127 (SEQ ID NO: 3) and SG128 (SEQ ID NO: 4) primer pair (Table 2) and cloned in pFpV25 vector digested with the BamHI and NdeI restriction enzymes. The p*lop1*-GFP M90T and p*lop1*-GFP K12 constructs were obtained and sequenced (Table 1).

In order to express *lop1-mCherry,* the *lop1* gene and its promoter (≈500bp) were amplified with the SG154 (SEQ ID NO: 27) and SG155 (SEQ ID NO: 14) primer pair (Table 2) and cloned in pSUC vector digested with the HindIII and XbaI restriction enzymes. The K84A point mutation of *lop1* was performed using the SG114 (SEQ ID NO: 5) and SG115 (SEQ ID NO: 6) primer pair (Table 2). The truncation of the N-terminal part of *lop1* (Δ1-59) was performed using the SG164 (SEQ ID NO: 15) and SG165 (SEQ ID NO: 16) primer pair (Table 2). Both mutated version of *lop1* were amplified with the SG154 (SEQ ID NO: 27) and SG155 (SEQ ID NO: 14) primer pair (Table 2) and cloned in pSUC vector digested with the HindIII and XbaI restriction enzymes. Respectively the p*lop1*-mcherry, p*lop1*_{K84A}-mCherry and p*lop1*Δ₁₋₅₉-mCherry constructs were obtained and sequenced (Table 1).

In order to control the expression of *lop1*-mCherry, *lop1*_{K84A}-mCherry and *lop1*Δ₁₋₅₉-mCherry (from *lop1* start codon) with a *lacI* promoter, the corresponding fragments were amplified from the p*lop1*-mCherry, p*lop1*_{K84A}-mCherry the SG278/SG329 (SEQ ID NOs: 17 & 19) primer pair (Table 2) and from the p*lop1*Δ₁₋₅₉mCherry constructs with the SG328/SG329 primer pair (SEQ ID NO: 18 & 19) (Table 2) and cloned in pSU19 digested with the BamHI and the EcorI restriction enzymes. The pSU-*lop1*-mCherry, pSU-*lop1_{K84A}*-mCherry and pSU-lop1Δ₁₋₅₉-mCherry constructs were obtained and sequenced (Table 1).

In order to overproduce the Lop1-H₆, Lop1_{K84A}-H₆, Lop1Δ₁₋₅₉-H₆ and Lop1₁₋₅₉-H₆ protein fusions in an IPTG-dependent manner the corresponding *lop1* DNA fragment were amplified by PCR prior cloning in the pKJ1 plasmid, digested at the NcoI and BamHI restriction (Table 1). *lop1* was amplified using the SG90/SG91 (SEQ ID NOs: 7 & 8) primer pair (Table 2), *lop1_{K84A}* was obtained using the SG150/1G151 primer pair (SEQ ID NO: 11 & 12) to introduce a single point mutation K84A (Table 2). *lop1*Δ₁₋₅₉ was amplified using the SG157/SG91 (SEQ ID NO: 9 & 8) primer pair (introducing an additional Methione at the N-terminus) (Table 2) and *lop1*₁₋₅₉ was amplified using the SG90/SG169 primer pair (SEQ ID NO: 7 & 10) (introducing a 5' stop codon in the ORF) (Table 2). The resulting p*lop1*-H₆, p*lop1_{K84A}*-H₆, p*lop1Δ₁₋₅₉*-H₆ and p*lop1₁₋₅₉*-H₆ constructs were analyzed by PCR and sequenced. In order to generate the H₆-Lop1Δ₁₋₅₉ and H₆-Lop1Δ₁₋₅₉Δ₃₀₃₋₃₇₅ constructs, sub-cloning was performed using LIC-cloning methodology, allowing the generation of the pNIC28-Bsa4-*lop1Δ₁₋₅₉ and* pNIC28-Bsa4-*lop1Δ₁₋₅₉Δ₃₀₃₋₃₇₅* constructs.

### Rabbit ligated ileal loop model

New Zealand White rabbits weighting 2.5-3 kg (Charles River Breeding Laboratories, Wilmington, MA) were used for experimental infections. For each animal, up to 12 intestinal ligated loops, each 5 cm in length, were prepared as described previously (Martinez et al., 1988; West et al., 2005). For the evaluation of the C.I., an equal quantity of the wild-type strain and of the mutant was injected in each loop (corresponding to a total dose of 10⁵ CFU per loop). After 16h, animals were sacrificed and the luminal fluid was aspirated and *S. flexneri* recovered. C.I. was calculated as the proportion of mutant to wild-type bacteria recovered from animals, divided by the proportion of mutant to wild-type in the inoculums, and results are expressed as the mean of at least 4 loops from two independent animal. The experimental protocol was approved by the Ethic committee Paris 1 (number 20070004, December 9th 2007).

For immunohistochemical staining, infected rabbit ileum samples were washed in PBS, incubated at 4°C PBS containing 12% sucrose for 90 min, then in PBS with 18% sucrose overnight, and frozen in OCT (Sakura) on dry ice. 7 µm sections were obtained using a cryostat CM-3050 (Leica). Fluorescent staining was performed using a rabbit anti-*Shigella* LPS primary antibody (1:200 dilution) (P. Sansonetti, Institut Pasteur) and an anti-rabbit-FITC conjugated secondary antibody (1:1000). Epithelium cell nuclei were stained with Dapi (1:1000) and actin stained with RRX-Phalloidin (1:1000). Image acquisition was performed using laser-scanning confocal microscopy. Image analysis was performed using ImageJ software.

### Two hybrids screen

The inventors used the BACTH system that is based on the interaction-mediated reconstitution of an adenylate cyclase (AC) enzyme in the otherwise defective *E*. *coli* strain DHM1 (Valdivia and Falkow, 1996; Karimova et al., 1998). This system is composed of two replication compatible plasmids, pKT25 and pUT18, respectively, encoding the intrinsically inactive N-terminal T25 domain and C-terminal T18 domain of the AC enzyme. *E. coli* and *Shigella lop1* was amplified using the NG1281 and NG1282 primer pair (SEQ ID NO: 1 & 2) and cloned in pKT25 vector.

pKT25 and pUT18 plasmids, which were subsequently doubly transformed to DHM1 to search for AC reconstitution that turns on β-galactosidase production leading to the blue colour after 2 days of growth at 30°C on indicator plates containing Xgal (Eurobio, 40 mg ml-1), isopropyl-1-thio-b-D-galactopyranoside (Invitrogen, 0.5 mM), Ap, Km and nalidixic acid. β-galactosidase activity was measured as described before, averaged from three independent experiments and expressed as Miller Unit (Sansonetti et al., 1982; Karimova et al., 1998).

### Antibody

*α-Lop1 antibody production.* An α-Lop1 rabbit polyclonal antibody was collected from two New-Zealand rabbits challenged with purified Lop1-H₆ (2 mg/mL solution) on four occasions with the purified protein separated by 2-weeks rest. The first injection (500 µL, intradermal) was performed with the purified protein (125 µg) in addition with a complete Freund's adjuvant. The second injection was performed following a similar procedure in the presence of incomplete Freund's adjuvant. The third and the last injection were performed with no adjuvant. Final blood collection was performed by cardiac puncture in heparin-free tube. Sera were separated from blood cells by centrifugation (14000 rpm, 30 min). As a note, the α-Lop1 antibody obtain following this procedure allow the detection of Lop1-H₆ but also the Lop1_{K84A}-H₆ or Lop1Δ₁₋₅₉-H₆ mutated versions of Lop1-H₆ by western blot.

α-FtsZ rabbit polyclonal antibody was kindly provided by Pr. Kenn Gerdes (Weiss et al., 1999; Galli and Gerdes, 2010).

### Thin Layer Chromatography (TLC) analysis

ATPase and GTPase assays were performed in the presence of BSA 1.25 mg/mL (Sigma), ATPγ32 or GTPγ32 30 µCi (Perkin Emer), ATP or GTP 50 mM (Sigma) and 0.1 to 10 µg of purified Lop1-H₆ and Lop1_{K84A}-H₆ as indicated. The final reaction mixture volume is 20 µL in a TMD buffer (Tris pH7.4 25 mM, MgCl₂ 10 mM, DTT 1 mM). The reaction was run during 10 min at 37°C and stopped by the addition of 20 µL methanol. When indicated, the chromatography is performed on TLC plates (Thomas Scientific), with a mobile phase containing a mixture of lithium chlorure (LiCl) and of formic acid. After migration, a film is exposed on the plate and further developed. Radiolabelled Pi presence through ATPg32 hydrolysis is then revealed.

### ATPase assay

The ATPase assay was performed using the colorimetric Pi ColorLock ALS kit (Innova Biosciences) in the presence of 5 µmol Lop1 at 37°C. A₅₉₅ₙₘ absorbance measurements were performed at t=2 min. The reactions occurred at 37°C during 2 min in a TrisHCl 50 mM pH=7.4 buffer containing 5 mM MgCl₂. The experimental data (Vmax, Km) were analyzed with the Michaelis-Menten equation, using a nonlinear regression analysis program (Kaleidagraph, Synergy Software) on three independent experiments performed as duplicate.

### Western blot analysis

Western blot analyses were performed either on bacterial extracts or on purified proteins (polymerization and proteolysis assays).

Bacterial extracts were prepared as followed. For FtsZ and Lop1 detection in K12 and K12::Δ*lop1* strains, overnight bacterial cultures were subcultured in 100 ml LB liquid media at 37°C until the O.D. A₆₀₀ reached 0.3. Bacteria were harvested by centrifugation for 15 min at 3,000 *x* g, washed, then re-suspended in 10 ml PBS. Cells were spun again for 5 min at 3000 *x* g, and re-suspended in 10 mL of PBS. Membranes associated (Memb.) and cytosolic (Cyt.) proteins were separated by centrifugation for 20 min at 12,000 x g.

For FtsZ and Lop1 detection in the K12Δ*lop1* strain upon Lop1-H₆ and mutated versions overexpression (p*lop1*-H₆, p*lop1_{K84A}*-H₆, p*lop1Δ₁₋₅₉*-H₆) overnight bacterial cultures were subcultured in 800 ml LB liquid media at 37°C in the presence of IPTG at a final concentration of 1 mM for 3h. As indicated, bacteria were harvested by centrifugation for 15 min at 3,000 x g, washed, then subcultured in an equal volume of fresh LB liquid media. For each time point (0, 30 and 60 min), 100 mL of bacterial culture were harvested by centrifugation for 15 min at 3,000 x *g*, washed, then re-suspended in 10 mL PBS for sonication. Membranes associated (Memb.) and cytosolic (Cyt.) proteins were separated by centrifugation for 20 min at 12,000 *x g*. For FtsZ and Lop1 detection in polymerization and proteolysis assays, 100 µL of the reaction mixture are loaded on each well.

Total protein concentrations were measured by the method of Bradford (Biorad). Proteins were separated by 16% SDS-PAGE and transferred to nitrocellulose membranes, and incubated with the primary antibodies diluted in PBS/5% milk/0.01% Tween20 (Sigma) overnight. Membranes were washed in PBS three times, then incubated with secondary antibodies for 1 hour before washing. Antibody binding was detected with chemiluminescence (ECL kit, GE Healthcare).

### Electron-microscopy analysis

*Bacteria.* MG1655 *E. coli* wild-type and Δ*lop1* strains were observed by EM for immunodetection of Lop1. For immuno-EM, bacteria were fixed with 4% formaldehyde in 0.1 M phosphate buffer (pH 7.4), and embedded in 12% gelatin. Blocks were infiltrated with 2.3 M sucrose for cryoprotection, mounted on specimen holders and frozen in liquid nitrogen. Cryosections were performed with a Leica EM UC6/FC6 Microtome (Leica Microsystems, Vienna, Austria). A labeling was performed on thawed cryosections using antibody directed against Lop1, which is recognized by protein A gold. Cryosections were labeled first with an α-Lop1 rabbit polyclonal antibody at 1/1000 dilution, then with protein-A gold-10 nm diluted at 1/60 obtained from Utrecht University (Utrecht, The Netherlands) (Slot et al., 1991; West et al., 2005). The grids were viewed on a Jeol JEM 1010 (Japan) transmission electron microscope at 80 kV and Images were taken using a KeenView camera (Soft Imaging System, Lakewood, CO, USA) using iTEM5.0 software (Soft Imaging System GmbH).

*Polymerized proteins.* FtsZ and Lop1 protein extracts were negatively stained with 2% uranyl acetate on glow discharged copper grids. The samples were observed in a Jeol 1200EXII or a JEM 1010 microscope (Jeol Company, Tokyo Japan) at 80-kV with an Eloise Keenview camera. Images were recorded with Analysis Pro Software version 3.1 (Eloise SARL, Roissy, France).

### Bioinformatics

Lop1 homologous proteins identification among other organisms was performed using BlastP. The Lop1 sequence comparisons were performed using the ClustalW software. Bacteria length measurement was performed with the MicrobeTracker suite software (version 0.937) (Weiss et al., 1999; Sliusarenko et al., 2011) and the data mining was performed using the Matlab computing system (R2012 version with Image Processing Toolbox and Statistics Toolbox). Statistical analyses were performed using the Graphpad Prism 5 software.

### Fluorescent protein-fusions imaging

In order to localize FtsZ-GFP and Lop1-mCherry and mutated versions protein fusions in bacteria, the corresponding expression plasmids were transformed in *E*. *coli* K12 MG1655 wild-type or Δ*Lop1* strains, as indicated. The localization was either performed on fixed or living bacteria. The fixation of bacteria was performed by adding 4% PFA followed by a washing in PBS. The observation was performed using a Nikon Eclipse 80i epifluorescent microscope. The live observation of FtsZ-GFP and Lop1-mCherry during the cell division process was performed on LB agar (1%) pad using a 200M Axiovert epifuorescent microscope (Zeiss) equipped with a Lambda LS 300W Xenon lamp and a CoolSnapHQ CCD camera.

### Example 2 - RESULTS

### Identification of lop1, essential for ileal loop colonization by Shigella

The inventors identified *lop1* while screening a library of *Shigella flexneri* mutants by performing Signature Tagged Mutagenesis (STM, (Hensel et al., 1995; Raskin and de Boer, 1997),(Rothfield et al., 2005; West et al., 2005; de Boer, 2010) (Rothfield et al., 2005; de Boer, 2010; Marteyn et al., 2010)); a transposon insertion located 12 bp upstream of the predicted ORF was defective for gastrointestinal colonization and had a growth defect (not shown). The *lop1* gene is 1128 bp in length and the predicted proteins in *E*. *coli* K12 (accession number b3232, *yhcM*) and *Shigella* M90T (accession number S3484) share 97.6% amino acid identity and putative Walker A and Walker B sites (Figures 1A and 8A).

Lop1 is conserved among Gram-negative bacteria (cocci and bacilli). In addition, homologues with up to 27% amino acid identity can be found among the eukaryotic kingdom such as fungi (C. *albicans*), yeast (*S. cerevisiae*) or human (*H. sapiens*) (Figure 1A and Table 3), which are predicted to be localised in mitochondria, although no experimental demonstration has yet been provided.

### Loss of lop1 leads to a temperature-dependent filamentous phenotype.

*lop1* mutants were constructed in *E*. *coli* K12 strain MG1655 (K12::Δ*lop1*) and in *S. flexneri* strain M90T (M90T::Δ*lop1*), and complemented (K12::Δ*lop1*-p*lop1*-GFP and M90TΔ*lop1*-p*lop1*-GFP respectively). The *S. flexneri lop1* mutant was attenuated for GI colonization (Figure 8B) with a reduced tissue destruction compared with the wild-type strain (Figure 8C). Of note, M90T::Δ*lop1* had a filamentous phenotype *in vivo* (Figure 8D).

*In vitro*, the K12::Δ*lop1* and M90T::Δ*lop1* mutants had a temperature-dependent elongated phenotype as compared to wild-type strains. At 42°C, average bacterial cell length increased significantly from 4.6±0.9 to 5.3±3 µm in K12::Δ*lop1* and 4.6±0.9to 93±18µm in M90T::Δ*lop1* (Figures 1B, 1C, 1D, Student's T test p<0.01 or p<0.001) and was abolished by complementation of the mutants (Figure 1B, 1C and 9A). Interestingly, the conditional elongation phenotype could be complemented by eptotic expression of *lop1-GFP* in both strains suggesting that the GFP-fusion was functional (Figure 1B and 1C). The avirulent M90T strain INV-::Δ*lop1* (virulence plasmid cured, congo-red negative strain) showed an intermediate filamentous phenotype as the mean length of bacteria was reduced as compared to M90T::Δ*lop1* at 42°C (18±3 µm vs 93±18 µm, p<0.001, Figures 1B and 9A). As a general statement, the temperature-dependent elongated phenotype was observed in all cases, comparing growth at 30°C and 42°C (Student's T test p<0.001) (Figures 1D and 9A).

### Lop1 is an ATPase conserved among Gram-negative bacteria, which undergoes an ATP-dependent autoproteolysis.

To determine the biochemical function of Lop1, due to the presence of a putative nucleotide-binding site (GGVGRGK₈₄T), the inventors tested its ability to hydrolyse ATP and GTP. They first observed that Lop1 is a monomeric protein by gel filtration (data not shown). The inventors demonstrated *in vitro* that purified K12 Lop1-H₆ hydrolyses ATP but not GTP (Figure 1D). Point mutation of the putative Walter-A ATP-binding site (Figure 9A, GGVGRGKT) abolished ATPase activity (Figure 9B). Furthermore, in the presence of ATP, Lop1 undergoes an ATP-dependent autolytic cleavage at amino acids L₅₉/W₆₀ confirmed by N-terminal sequencing analysis (Figure 1E). Cleavage was not detected with ATP and additional EDTA, with AMP-PNP instead of ATP, or with Lop1_{K84A} (Figure 9C). The inventors observed that Lop1Δ₁₋₅₉ is a monomeric protein in solution (data not shown). As Lop1Δ₁₋₅₉ is generated upon reaction of Lop1 with ATP, the enzymatic parameters (Km, Vmax) of the full-length protein could not be calculated. Alternatively, the inventors could determine Lop1Δ₁₋₅₉ Km=0.25±0.1 mM and Vmax= 7.98 ± 0.87 mM Pi.min⁻¹ (Figure 9D). Interestingly, the generation of Lop1Δ₁₋₅₉ was associated with the assembly of protein polymers observed by negative stain electron-microscopy (Figure 1F). In addition, Lop1Δ₁₋₅₉ alone was able to associate as polymers in the presence of ATP, indicating that the N-terminal fragment is not required for polymerization (Figure 1F). In addition, incubating Lop1 in the presence of ATP the inventors could isolate polymers by ultracentrifugation and demonstrate that they are composed of Lop1 and Lop1Δ₁₋₅₉, as Lop1 only remained in the soluble fraction and Lop1Δ₁₋₅₉ accumulates in the pellet fraction (Figure 1G).

### E. coli Lop1 interacts with FtsZ in vitro.

To further define role of Lop1 the inventors searched for potential interactions between Lop1 and components of the divisome. Using the BATCH two-hybrid system (Karimova et al., 1998; 2005; Adams and Errington, 2009), they found interactions between Lop1 and FtsQ, FtsL, FtsI and FtsN respectively (Student's T test p<0.01). No interaction was observed with FtsA, ZipA and FtsK. A similar result was obtained using Lop1 from *S. flexneri* (M90T) as a bait (Figure 2A). Interactions with FtsZ could not be tested using the two-hybrid system, as previously described (Mukherjee and Lutkenhaus, 1994; Karimova et al., 2005).

The interactions were confirmed by pulldown. FtsQ-GFP (pDSW240), FtsL (pDSW326), FtsI-GFP (pDSW234) and FtsN-GFP (pDSW238) from *E. coli* lysates with and interact with Lop1 bound to beads. Although no interaction was observed with ZipA (Figure 2B). An interaction between Lop1 and FtsZ-GFP (pDSW230) was detected (Figure 2C). This interaction is dependent of the N-terminal region of Lop1 but not its ATP-binding site (Figure 2C).

Next, the cellular localization of divisome components was analysed in the absence of Lop1 (K12::Δ*lop1*). Loss of Lop1 resulted in multiple FtsZ-rings along filamentous bacteria (Figure 2D), while the localization of FtsQ, FtsL and FtsN was affected by the absence of Lop1 (Figure 10), suggesting that Lop1 recruitment was downstream of Z-ring maturation step. This phenotype was more marked during the growth in rich media (LB) and at higher temperatures (Figure 2E) or during stationary phase (Figure 11A), consistent with the temperature-dependent phenotype seen in the K12::Δ*lop1* mutant (Figure 1C and 1D). Eventually, the filamentation of the K12::Δ*lop1* strain expressing FtsZ-GFP (pDSW231) was observed using live fluorescent microscopy (Figure 11B).

FtsZ-GFP is not fully functional but does not impair the cell division process when expressed at a basal level, tolerated by K12 wild-type strain (Oliva et al., 2004; Thanedar and Margolin, 2004). However, the observation of dysfunctional Z-rings associated with the formation of filamentous bacteria in K12::Δ*lop1* expressing FtsZ-GFP suggests a role of Lop1 in the control of Z-ring dynamics.

Next, as aberrant cell division phenotype was observed in the K12::Δ*lop1* mutant, the localisation of Lop1 and FtsZ in the K12 wild-type and K12::Δ*lop1* mutant strains was analysed by Western blot using rabbit polyclonal antibodies. The inventors demonstrated that Lop1 was a cytosolic protein as FtsZ was found in cytosolic and membrane fractions (Figure 2F) as described previously in dividing bacteria (Shlomovitz and Gov, 2009) and modelled in liposomes (Osawa et al., 2009). The localisation of Lop1 was further confirmed by EM analysis using a polyclonal anti-Lop1 associated with an immunogold staining which allowed the detection of Lop1 predominantly cytoplasmic, in the close vicinity of the bacterial inner membrane (Figure 2G), which is consistent with its ability to interact with FtsZ.

### Lop1 recruitment co-localises with constricting Z-rings in vivo. The inactivation of lop1 leads to an accumulation of immature Z-rings.

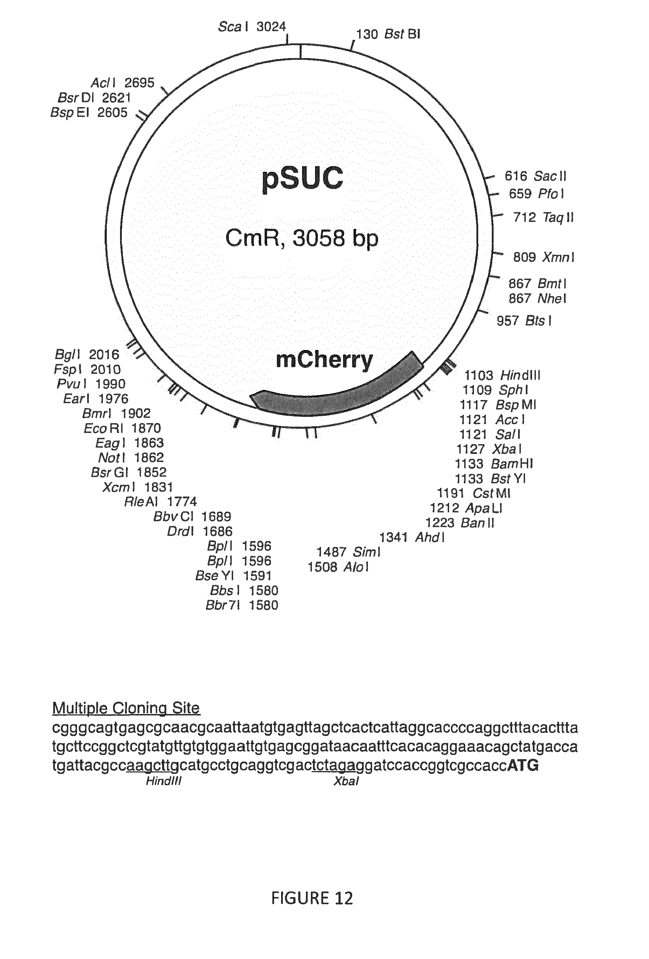

In order to further examine the relationship between Lop1 and FtsZ during cell division, the inventors performed timelapse observations with *E*. *coli* K12::Δ*lop1* expressing Lop1-mCherry under the *lop1* promoter control (pSUC plasmid, Figures 12) and FtsZ-GFP (pDSW230) (Weiss et al., 1999) without IPTG, allowing a basal level of FtsZ-GFP expression. By live microscopy, the inventors demonstrated the recruitment of Lop1-mCherry at the Z-ring at late stage of cell division (Figure 3A). This recruitment of Lop1-mCherry was correlated with a decrease of the Z-ring diameter (Figures 3B and 3C), while the Lop1-mCherry signal was co-localizing with the FtsZ-GFP signal at each time point (Figure 3B, n=5 represents five individual observations extracted from three independent experiments).

Considering the maximum of Z-ring constriction as a final state, these quantifications were performed at -50min, -15min, -10min, -5min. As compared to the -50min time point (before Maximum constriction), the level of the Lop1-mCherry signal and the diameter of the Z-ring were inversely correlated at the -15min, -10min, -5min time points and at the Maximal constriction (Max. const.) (Figure 3C, Student's T test p<0.001). In the absence of Lop1-mCherry, no restriction of the Z-ring was observed (Figures 3D and 11B). Lop1 lacking residues 1-59 was not associated with the Z-ring (Figure 3E), which was consistent with the inability of Lop1Δ₁₋₅₉ to interact with FtsZ *in vitro* (Figure 2C).

### Overexpression of Lop1 induces bacterial filamentation and Z-ring disruption in vivo. Lop1 is processed in vivo into Lop1Δ₁₋₅₉.

Different versions of Lop1 were constitutively expressed to further study the influence of this protein on Z-ring stability and constriction.

Overexpression of the Lop1 protein induced the formation of twisted filamentous bacteria (Figure 4A and 4B), while overexpressing Lop1_{K84A} and Lop1Δ₁₅₉ had no consequence on the bacterial shape (Figure 4B). Overexpression of the Lop1 59aa N-terminal fragment only (Lop1₁₋₅₉-6xHis) was toxic for the bacteria (data not shown). Reducing the IPTG dose to 0.1 mM induced the formation of filamentous bacteria, suggesting that this fragment interfered with FtsZ (Figure 4B).

In order to evaluate the level of Lop1 turnover, overexpression of Lop1 was performed at a lower level and stopped by eliminating IPTG form the media (t=0 min). At t=0 the inventors could recapitulate the results described in Figure 4B. The inventors could then observe at t=30 min and t=60 min that the filamentous phenotype associated to Lop1 overexpression was reversed. In controls (Lop1_{K84A} and Lop1Δ₁₋₅₉) the bacterial shape remained normal as compared to the wild-type at all time points (Figure 4C). As observed previously *in vitro*, the ATP-dependent cleavage of Lop1 (Figure 1E), was recapitulated by Western blot using an anti-His antibody. Indeed, Lop1Δ₁₋₅₉ was detected in the pellet fraction upon Lop1 overexpression at t=60 min (Figure 4D), which was not observed upon Lop1_{K84A} overexpression (Figure 4D). These observations were confirmed using an anti-Lop1 antibody (Figures 4E). Interestingly, overexpression of Lop1-6xHis contributed to accumulation of an abnormally high level of FtsZ in the pellet fraction, while overexpressing Lop1Δ₁₋₅₉ caused a reduced amount of FtsZ polymers in the pellet fraction (Figure 4E).

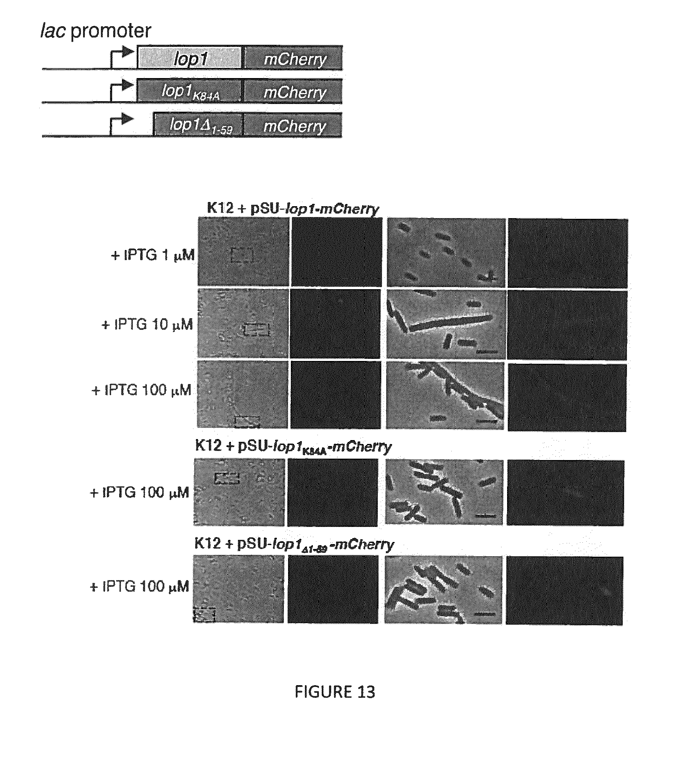

As Lop1 overexpression seemed to perturb the Z-ring constriction, the inventors aimed at visualising FtsZ polymerization in this condition. The FtsZ-GFP protein fusion was well tolerated by the *E. coli* wild-type strain, even though the fluorescent signal was low (Figure 5A), while it led to the formation of filamentous bacteria in the K12::Δ*lop1* mutant (Figure 5A), as previously observed (Figure 2D). Subsequently, the overexpression of Lop1-mCherry perturbed Z-ring constriction in a dose-dependent manner (Figure 5B), while the overexpression of Lop1_{K84A}-mCherry or Lop1Δ₁₋₅₉-mCherry did not lead to either bacterial shape modification (Figure 5B). For unclear reasons, in the presence of pJC104, the Lop1-mCherry signal was undetectable (data not shown) as compared to the wild-type strain in the absence of pJC104 (Figures 12B and 13).

### Fluorescence-based assay of FtsZ proteolysis

In order to confirm the FtsZ polymer proteolysis by Lop1Δ₁₋₅₉, the inventors designed a fluorescence-based assay as polymerization of a FtsZ/FtsZ-GFP mixture leads to an increase of the solution fluorescence (Trusca and Bramhill, 2002). In the presence of GTP, purified FtsZ and FtsZ-GFP form polymers, as described previously (Yu and Margolin, 1997b); however the level of the detected fluorescence remains low and the polymers length was reduced (Figures 17A and 17B uppermost row). In the presence of Lop1Δ₁₋₅₉, the inventors observed a rapid and significant increase of the fluorescence level (Figure 17A), which correlates with the formation of an homogeneous FtsZ/FtsZ-GFP polymer network (Figure 17B middle row, t=0). Interestingly, these polymers were rapidly degraded (Figure 17B middle row, t=10min), which correlated with a significant decrease of the fluorescence level (Figure 5C, Student's T test p<0.01). As a control, the simultaneous addition of benzamidin with Lop1Δ₁₋₅₉ did not impair the fluorescence increase (Figure 17B lowermost row), although preventing the FtsZ/FtsZ-GFP polymers degradation (Figure lowermost row), in association with a stable level of the fluorescent signal (Figure 17A). Interestingly, the addition of full length Lop1 led to a reduced fluorescence increase (Figure 17A), led to the formation of large and bundled polymers of FtsZ/FtsZ-GFP, which remained stable over time (Figure 17A).

### Lop1Δ₁₋₅₉ proteolyses FtsZ polymers in vitro.

To determine whether Lop1 acts directly on FtsZ polymers, the inventors performed FtsZ polymerization assays in the presence of purified proteins (Lop1, Lop1_{K84A} and Lop1Δ₁₋₅₉); the soluble and polymerized forms of FtsZ were separated by ultracentrifugation. FtsZ formed polymers in a GTP-dependent manner in 3 min at 30°C (Figure 14).

The FtsZ polymers incubation with Lop1 lead to their slight degradation at t=3min in an ATP-dependent manner (Figure 6A), with no obvious increase in soluble FtsZ level suggesting a proteolysis of FtsZ by Lop1. In contrast, no degradation of FtsZ polymers was observed with Lop1_{K84A} (Figure 6A). Strikingly, incubation of FtsZ polymers with Lop1Δ₁₋₅₉ in the absence of ATP caused its significant degradation (t=1min, no FtsZ signal in the soluble fraction), in association with a complete degradation of Lop1Δ₁₋₅₉ (Figure 6A). In turn, the inventors hypothesize that the late initiation of FtsZ polymers proteolysis by Lop1 in the presence of ATP might be due to the formation of small amount of Lop1Δ₁₋₅₉-6xHis, as described above.

This result was confirmed by TEM analysis of FtsZ polymers (Yu and Margolin, 1997a; Adams and Errington, 2009) (Mukherjee and Lutkenhaus, 1994; Mateos-Gil et al., 2012), as the simultaneous addition of Lop1 with ATP or Lop1Δ₁₋₅₉ in the absence of ATP induced a disruption or complete degradation of FtsZ polymers respectively (Figure 6B). Conversely, no effect was observed in the presence of Lop1 with ATP and additional EDTA or Lop1Δ₁₋₅₉ with PMSF (Figure 6B). This result was consistent with the FtsZ polymers degradation activity of Lop1-Δ₁₋₅₉ and confirmed the ATP-dependent proteolysis of FtsZ by Lop1 and indirectly the ATP-dependent autoproteolysis of Lop1 (Figure 2F). This experiment showing an inhibition of the proteolytic activity of Lop1Δ₁₋₅₉ by PMSF suggested that this protein has a serine protease activity.

In a strain expressing Lop1-mCherry and FtsZ-GFP, the inventors observed that a small population of bacteria (1.5%(±2), n=234) expressed a strong Lop1-mCherry signal (Figure 6C), as most of the bacterial population was associated with a Z-ring (89%(±7), n=234) (Figure 6C). In the whole population, the Lop1-mCherry signal and Z-ring detection were found to be exclusive (Student's T test p<0.001, n=232). In contrast, Lop1_{K84A}-mCherry or Lop1Δ₁₋₅₉-mCherry positive cells showed no such effect on Z-rings (Figure 15). Focusing on Lop1-mCherry positive cells for up to 120 min of growth, no Z-ring could be detected and over the time, as they did not divide (Figure 6D). These observations support the previous results showing a Lop1-dependent degradation of Z-ring in *E*. *coli.*

The inventors demonstrated that Lop1Δ₁₋₅₉ proteolytic activity was independent from the ClpX/ClpP complex which was recently described as being involved in FtsZ proteolysis *in vitro* (Camberg et al., 2009) (Oliva et al., 2004; Sugimoto et al., 2010) (Romberg and Mitchison, 2004; Camberg et al., 2011). Briefly, through a gel-filtration analysis, the inventors demonstrated that Lop1 1 in the presence of AMP-PNP was not forming complex with ClpP *in vitro* (Figure 16A and 16B). As a confirmation of this result, Lop1 is a monomeric protein *in vitro,* in contrast ClpX and ClpP organised as a proteolytic hexameric complex *in vitro* (Mukherjee and Lutkenhaus, 1998; Maillard et al., 2011).

As a final experiment, in order to confirm that Lop1Δ₁₋₅₉ was a serine protease, the inventors repeated the FtsZ polymers proteolysis assay in the presence of Lop1Δ₁₋₅₉ and various protease inhibitors. The inventors found that serine protease inhibitors (PMSF and leupeptin) inhibited the proteolysis of FtsZ polymers, whereas the the presence of EDTA or pepstatin had no effect (Figure 6E). Indeed the inventors found that the 73aa C-terminal fragment of Lop1Δ₁₋₅₉ was required for the proteolytic degradation of FtsZ polymers (Figure 6F).

### DISCUSSION

In this work, the inventors have characterized Loopin1 (Lop1), an ATPase conserved among Gram-negative bacteria. Lop1 was named in relation to its function as the inventors present evidence that this protein has a "looping effect" on the Z-ring, allowing its constriction and the cell division process to end (Figure 3A).

Lop1 was not an obvious candidate to play a role in the cell division control. First, *lop1* is neither an essential gene in *E*. *coli* nor in *Shigella,* so the initial screens aiming at identifying essential cell division genes missed *lop1.* Second, when fluorescent fusions (mCherry or GFP) of Lop1 are expressed in *E*. *coli*, no recruitment at the Z-ring is observed (Figures 6C and 6D), as classically reported for most of the divisome complex components (Stricker et al., 2002; de Boer, 2010). The inventors could observe a dynamic recruitment of Lop1-mCherry at the Z-ring using recent imaging technologies such as live microscopy (Figure 3A and Movie S1). To further Lop1 function, the inventors demonstrated that it autoproteolyses in an ATP-dependent manner, generating a N-terminal truncation (1-59) leading to the production of Lop1Δ₁₋₅₉ (or tLop1, Figure 7) (Figure 1E). Lop1Δ₁₋₅₉ is a serine protease (Figure 6E) and its C-terminal part (between aa 303-375) is required for its activity (Figure 6F).

### Lop1Δ₁₋₅₉ is an active serine protease generated from Lop1 through an ATP-dependent autoproteolytic process

The inventors identified FtsZ as a target of Lop1 and shown that the N-terminal fragment of Lop1 is not essential for its ATP-dependent polymerization (Figure 1F), but is required for the interaction between Lop1 and FtsZ, which has been observed *in vitro* using an His-pulldown approach (Figure 2B). This result was confirmed using a live microscopy approach, allowing the visualisation of Lop1 recruitment during the constriction of the Z-ring (Figure 3A). The inventors showed that Lop1 recruitment occurs downstream of the Z-ring maturation, as the localization of FtsQ, FtsL and FtsN are not impaired in the absence of Lop1 (Figure 11A). Indeed, the inventors have demonstrated that the accumulation of Lop1 at the Z-ring correlated to its constriction (Figure 4B, 4C and 4D). As the results show *in vitro* that the Lop1Δ₁₋₅₉ truncated form proteolyses FtsZ polymers (Figure 6B), the inventors speculate that this activated form of Lop1 might be generated upon interaction with FtsZ during the cell division (Figure 7). This hypothesis is supported by the observation that generation of Lop1Δ₁₋₅₉ is required for the formation of Lop1/Lop1Δ₁₋₅₉ mixture composed filaments (Figure 1F), which are observed during the cell-division process (Figure 3A).

The ATP-dependent autoproteolytic activation of Lop1 is comparable to the subtilisin (serine protease) autoproteolytic maturation consisting in a 77 aa prodomain processing (Bryan et al., 1995). In analogy with the subtilisin catalytic triade (Asp-32, His-64, and Ser-221), the Lop1 active site will have to be characterized in details through a mutagenesis approach and structural analysis.

Until this work, the regulation mechanism by which a Z-ring will initiate its constriction has been a controversial question. A first model suggested that FtsZ polymers could mediate their own constriction through GTP hydrolysis leading to their depolymerization (Scheffers and Driessen, 2001). Another model suggested that MAP-like proteins to be identified could be recruited at the Z-ring to modulate FtsZ polymers stability, bundling and disassembly (Adams and Errington, 2009). Only recently, the hypothesis of a proteolysis-dependent control of the Z-ring constriction emerged.

### Z-ring proteolysis is associated with its constriction

It has been reported previously in *Caulobacter crescentus* that the rate of FtsZ degradation increases after the initiation of the cell division, leading to a decrease of the Z-ring diameter (Kelly et al., 1998) however the underlying mechanism was not described. This proteolytic model of FtsZ constriction was supported by recent observations describing the proteolytic degradation of FtsZ polymers by the ClpX (ATPase)/ClpP (protease) hexameric complex (Hensel et al., 1995; Camberg et al., 2009) (West et al., 2005; Sugimoto et al., 2010) (Camberg et al., 2011), although no direct recruitment at the Z-ring of this complex was observed during a cell division process. Interestingly, the Lop1 N-terminal fragment has a role in the FtsZ interaction (Figures 2B and 3E) which can be compared to the N-terminal (65aa) domains of ClpX, which is also involved in the recognition of its substrate. The overexpression of the ClpX N-terminal fragment (but also the full length protein) cause filamentation and perturbates the Z-ring constriction (Karimova et al., 1998; 2005; Glynn et al., 2009) (Karimova et al., 2005; Sugimoto et al., 2010), as observed for Lop1 (Figure 4B). Indeed, in the absence of the N-terminal 59aa, Lop1Δ₁₋₅₉ did not interact with FtsZ (Figure 2B). Its overexpression no longer perturbates any longer the FtsZ polymerization (Figures 4B).

As *lop1* is not an essential gene in *E*. *coli* or in *Shigella,* it is still unclear to which the extent of Lop1 redundancy as the ClpX/ClpP complex and putatively other proteases provide FtsZ depredatory functions. This hypothesis would be supported by the vital role played by the Z-ring constriction control in the cell division process and survival of bacteria. Indeed, to date five AAA+-containing proteolytic systems have been identified in bacteria in general and more particularly in *E*. *coli* that are ClpX/P, ClpA/P, HslU/V, FtsH and Lon (reviewed in (Thanedar and Margolin, 2004; Hanson and Whiteheart, 2005)), which could putatively be involved in Z-ring proteolysis, as demonstrated for ClpX/ClpP *in vitro.* However, similarly to our observations concerning Lop1, their speculative dynamic recruitment at the Z-ring during the late stage of bacteria division will have to be demonstrated. This hypothesis is particularly true considering Gram-positive bacteria in which cell division process is controlled by FtsZ (as reviewed in (Errington et al., 2003; Shlomovitz and Gov, 2009) and (Goehring and Beckwith, 2005; Osawa et al., 2009)) but do not possess the *lop1* gene. Further study will deepen our knowledge on Z-ring constriction modulation in bacteria.

### Lop1 abundance regulation is critical for Z-ring constriction

Lop1 abundance regulation seems to be important for Z-ring constriction as the inventors show that the absence (Figure 12A) or the overexpression (Figure 4B) of *lop1* alters the cell division process through Z-ring constriction. In addition, our live microscopy allowed the detection of Lop1 cyclic accumulation at the Z-ring during each division (Figure 3A). Based on these observations, the inventors propose that Lop1 expression and degradation have to be tightly and cyclically controlled by the cell to allow functional Z-ring constriction and daughter cells separation processes. Our results show that Lop1 autoproteolyses in an ATP-dependent manner (Figure 1E) and that the FtsZ proteolysis by Lop1Δ₁₋₅₉ is associated with a degradation of the latter (Figure 6A). Our data suggest that the autoproteolysis of Lop1Δ₁₋₅₉ upon activation might be considered as a self-regulation of its proteolytic action. The question of a cyclic expression regulation of *lop1* is still unsolved. Addressing this question will provide information on the cell cycle control as it could be shown in the case of KiaC in the circadian clock control of cell division in cyanobacteria (Weiss et al., 1999; Dong et al., 2010).

The inventors propose that Lop1 plays a key role in the cell division process control through the proteolysis of FtsZ polymers. As the inactivation of *lop1* led to the loss of *Shigella* virulence *in vivo,* this protein should have to be considered as a novel putative antibiotic target for Gram-negative bacterial infection. Since the initial identification of the filamentous temperature sensitive (*fts*) genes and as highlighted by Beckwith and colleagues a decade ago (Buddelmeijer and Beckwith, 2002), the characterization of the whole set of proteins involved in the Z-ring-dependent bacterial division is still on going. It will be essential for a better comprehension of this key vital biological process.

### REFERENCES

Adams, D.W., and Errington, J. (2009). Bacterial cell division: assembly, maintenance and disassembly of the Z ring. Nat. Rev. Microbiol. 7, 642-653.

Benaroudj, N. et al., 2011. Assembly and proteolytic processing of mycobacterial ClpP1 and ClpP2. BMC biochemistry, 12, p.61.

Bi, E.F., and Lutkenhaus, J. (1991). FtsZ ring structure associated with division in Escherichia coli. Nature 354, 161-164.

Bryan, P., Wang, L., Hoskins, J., Ruvinov, S., Strausberg, S., Alexander, P., Almog, O., Gilliland, G., and Gallagher, T. (1995). Catalysis of a protein folding reaction: mechanistic implications of the 2.0 A structure of the subtilisin-prodomain complex. Biochemistry 34, 10310-10318.

Buddelmeijer, N., and Beckwith, J. (2002). Assembly of cell division proteins at the E. coli cell center. Curr. Opin. Microbiol. 5, 553-557.

Camberg, J.L., Hoskins, J.R., and Wickner, S. (2009). ClpXP protease degrades the cytoskeletal protein, FtsZ, and modulates FtsZ polymer dynamics. Proc. Natl. Acad. Sci. U.S.a. 106, 10614-10619.

Camberg, J.L., Hoskins, J.R., and Wickner, S. (2011). The interplay of ClpXP with the cell division machinery in Escherichia coli. J. Bacteriol. 193, 1911-1918.

Chen, J.C., Weiss, D.S., Ghigo, J.M., and Beckwith, J. (1999). Septal localization of FtsQ, an essential cell division protein in Escherichia coli. J. Bacteriol. 181, 521-530.

de Boer, P.A.J. (2010). Advances in understanding E. coli cell fission. Curr. Opin. Microbiol. 13, 730-737.

Datsenko, K.A., and Wanner, B.L. (2000). One-step inactivation of chromosomal genes in Escherichia coli K-12 using PCR products. Proc. Natl. Acad. Sci. U.S.a. 97, 6640-6645.

Dong, G., Yang, Q., Wang, Q., Kim, Y.-I., Wood, T.L., Osteryoung, K.W., van Oudenaarden, A., and Golden, S.S. (2010). Elevated ATPase activity of KaiC applies a circadian checkpoint on cell division in Synechococcus elongatus. Cell 140, 529-539.

Errington, J., Daniel, R.A., and Scheffers, D.-J. (2003). Cytokinesis in bacteria. Microbiol. Mol. Biol. Rev. 67, 52-65-tableofcontents.

Galli, E., and Gerdes, K. (2010). Spatial resolution of two bacterial cell division proteins: ZapA recruits ZapB to the inner face of the Z-ring. Mol. Microbiol. 76, 1514-1526.

Ghigo, J.M., Weiss, D.S., Chen, J.C., Yarrow, J.C., and Beckwith, J. (1999). Localization of FtsL to the Escherichia coli septal ring. Mol. Microbiol. 31, 725-737.

Glynn, S.E., Martin, A., Nager, A.R., Baker, T.A., and Sauer, R.T. (2009). Structures of asymmetric ClpX hexamers reveal nucleotide-dependent motions in a AAA+ protein-unfolding machine. Cell 139, 744-756.

Goehring, N.W., and Beckwith, J. (2005). Diverse paths to midcell: assembly of the bacterial cell division machinery. Curr. Biol. 15, R514-R526.

Hanson, P.I., and Whiteheart, S.W. (2005). AAA+ proteins: have engine, will work. Nat. Rev. Mol. Cell Biol. 6, 519-529.

Hensel, M., Shea, J.E., Gleeson, C., Jones, M.D., Dalton, E., and Holden, D.W. (1995). Simultaneous identification of bacterial virulence genes by negative selection. Science 269, 400-403.

Hu, Z., Mukherjee, A., Pichoff, S., and Lutkenhaus, J. (1999). The MinC component of the division site selection system in Escherichia coli interacts with FtsZ to prevent polymerization. Proc. Natl. Acad. Sci. U.S.a. 96, 14819-14824.

Karimova, G., Dautin, N., and Ladant, D. (2005). Interaction network among Escherichia coli membrane proteins involved in cell division as revealed by bacterial two-hybrid analysis. J. Bacteriol. 187, 2233-2243.

Karimova, G., Pidoux, J., Ullmann, A., and Ladant, D. (1998). A bacterial two-hybrid system based on a reconstituted signal transduction pathway. Proc. Natl. Acad. Sci. U.S.a. 95, 5752-5756.

Kelly, A.J., Sackett, M.J., Din, N., Quardokus, E., and Brun, Y.V. (1998). Cell cycle-dependent transcriptional and proteolytic regulation of FtsZ in Caulobacter. Genes Dev. 12, 880-893.

Maillard, R.A., Chistol, G., Sen, M., Righini, M., Tan, J., Kaiser, C.M., Hodges, C., Martin, A., and Bustamante, C. (2011). ClpX(P) generates mechanical force to unfold and translocate its protein substrates. Cell 145, 459-469.

Marteyn, B., West, N.P., Browning, D.F., Cole, J.A., Shaw, J.G., Palm, F., Mounier, J., Prevost, M.C., Sansonetti, P., and Tang, C.M. (2010). Modulation of Shigella virulence in response to available oxygen in vivo. Nature 465, 355-358.

Martinez, E., Bartolomé, B., and la Cruz, de, F. (1988). pACYC184-derived cloning vectors containing the multiple cloning site and lacZ alpha reporter gene of pUCB/9 and pUC 18/19 plasmids. Gene 68, 159-162.

Mateos-Gil, P., Paez, A., Hörger, I., Rivas, G., Vicente, M., Tarazona, P., and Vélez, M. (2012). Depolymerization dynamics of individual filaments of bacterial cytoskeletal protein FtsZ. Proc. Natl. Acad. Sci. U.S.a. 109, 8133-8138.

Mukherjee, A., and Lutkenhaus, J. (1994). Guanine nucleotide-dependent assembly of FtsZ into filaments. J. Bacteriol. 176, 2754-2758.

Mukherjee, A., and Lutkenhaus, J. (1998). Dynamic assembly of FtsZ regulated by GTP hydrolysis. Embo J. 17, 462-469.

Mukherjee, A., Cao, C., and Lutkenhaus, J. (1998). Inhibition of FtsZ polymerization by SulA, an inhibitor of septation in Escherichia coli. Proc. Natl. Acad. Sci. U.S.a. 95, 2885-2890.

Mukherjee, A., Saez, C., and Lutkenhaus, J. (2001). Assembly of an FtsZ mutant deficient in GTPase activity has implications for FtsZ assembly and the role of the Z ring in cell division. J. Bacteriol. 183, 7190-7197.

Oliva, M.A., Cordell, S.C., and Löwe, J. (2004). Structural insights into FtsZ protofilament formation. Nat. Struct. Mol. Biol. 11, 1243-1250.

Osawa, M., Anderson, D.E., and Erickson, H.P. (2009). Curved FtsZ protofilaments generate bending forces on liposome membranes. Embo J. 28, 3476-3484.

Pazos, M., Natale, P., and Vicente, M. (2012). A specific role for the ZipA protein in cell division: stabilization of the FtsZ protein. J. Biol. Chem.

Raskin, D.M., and de Boer, P.A. (1997). The MinE ring: an FtsZ-independent cell structure required for selection of the correct division site in E. coli. Cell 91, 685-694.

Romberg, L., and Mitchison, T.J. (2004). Rate-limiting guanosine 5'-triphosphate hydrolysis during nucleotide turnover by FtsZ, a prokaryotic tubulin homologue involved in bacterial cell division. Biochemistry 43, 282-288.

Rothfield, L., Taghbalout, A., and Shih, Y.-L. (2005). Spatial control of bacterial division-site placement. Nat. Rev. Microbiol. 3, 959-968.

Sansonetti, P.J., d'Hauteville, H., Formal, S.B., and Toucas, M. (1982). Plasmid-mediated invasiveness of "Shigella-like" Escherichia coli. Ann. Microbiol. (Paris) 133, 351-355.

Scheffers, D., and Driessen, A.J. (2001). The polymerization mechanism of the bacterial cell division protein FtsZ. FEBS Lett. 506, 6-10.

Shlomovitz, R., and Gov, N.S. (2009). Membrane-mediated interactions drive the condensation and coalescence of FtsZ rings. Phys Biol 6, 046017.

Sliusarenko, O., Heinritz, J., Emonet, T., and Jacobs-Wagner, C. (2011). High-throughput, subpixel precision analysis of bacterial morphogenesis and intracellular spatio-temporal dynamics. Mol. Microbiol. 80, 612-627.

Slot, J.W., Geuze, H.J., Gigengack, S., Lienhard, G.E., and James, D.E. (1991). Immuno-localization of the insulin regulatable glucose transporter in brown adipose tissue of the rat. J Cell Biol 113, 123-135.

Stricker, J., Maddox, P., Salmon, E.D., and Erickson, H.P. (2002). Rapid assembly dynamics of the Escherichia coli FtsZ-ring demonstrated by fluorescence recovery after photobleaching. Proc. Natl. Acad. Sci. U.S.a. 99, 3171-3175.

Sugimoto, S., Yamanaka, K., Nishikori, S., Miyagi, A., Ando, T., and Ogura, T. (2010). AAA+ chaperone ClpX regulates dynamics of prokaryotic cytoskeletal protein FtsZ. J. Biol. Chem. 285, 6648-6657.

Thanedar, S., and Margolin, W. (2004). FtsZ exhibits rapid movement and oscillation waves in helix-like patterns in Escherichia coli. Curr. Biol. 14, 1167-1173.

Trusca, D., Scott, S., Thompson, C., and Bramhill, D. (1998). Bacterial SOS checkpoint protein SulA inhibits polymerization of purified FtsZ cell division protein. J. Bacteriol. 180, 3946-3953.

Twining, S.S. Fluorescein Isothiocyanate Labeled Casein Assay for Proteolytic Enzymes Anal. Biochem. 143, 30-34(1984).

Valdivia, R.H., and Falkow, S. (1996). Bacterial genetics by flow cytometry: rapid isolation of Salmonella typhimurium acid-inducible promoters by differential fluorescence induction. Mol. Microbiol. 22, 367-378.

Weiss, D.S., Chen, J.C., Ghigo, J.M., Boyd, D., and Beckwith, J. (1999). Localization of FtsI (PBP3) to the septal ring requires its membrane anchor, the Z ring, FtsA, FtsQ, and FtsL. J. Bacteriol. 181, 508-520.

West, N.P., Sansonetti, P., Mounier, J., Exley, R.M., Parsot, C., Guadagnini, S., Prevost, M.C., Prochnicka-Chalufour, A., Delepierre, M., Tanguy, M., et al. (2005). Optimization of virulence functions through glucosylation of Shigella LPS. Science 307, 1313-1317.

Yu, X.C., and Margolin, W. (1997a). Ca2+-mediated GTP-dependent dynamic assembly of bacterial cell division protein FtsZ into asters and polymer networks in vitro. Embo J. 16, 5455-5463.

Yu, X.C., and Margolin, W. (1997b). Ca2+-mediated GTP-dependent dynamic assembly of bacterial cell division protein FtsZ into asters and polymer networks in vitro. Embo J. 16, 5455-5463.

## Claims

1. A method to identify substances which affect bacterial cell division by interfering with the function of LOP1, comprising the steps:
a) bringing into contact a purified protein selected from the group: FtsZ, FtsQ, FtsL, FtsI and FtsN; with purified LOP1 protein;
b) Assaying the formation of complexes between LOP1 and the selected other protein in the presence and absence of a substance to be tested;
c) Selecting substances from step b) which affect the formation of complexes when present.

2. The method according to claim 1, wherein in step:
a) FtsZ polymers are incubated with said LOP1 protein;
b) the degradation of said FtsZ polymers is assayed in the presence and absence of a substance to be tested;
c) selecting substances which when present in step b) affect the degradation of FtsZ polymers.

3. The method of claim 1 or 2 wherein said LOP1 protein is selected from the group: full length LOP1 (SEQ ID NO: 25) or a truncated version LOP1Δ₁₋₅₉ (SEQ ID NO: 26).

4. A method to identify substances which affect the autoproteolysis and/or ATP hydrolysis of LOP1, comprising the steps:
a) Incubating full length LOP1 with a substance to be tested in the presence and absence of ATP;
b) Monitoring the formation of LOP1Δ₁₋₅₉;
c) Selecting substances which when ATP is present in step b) decrease the formation of LOP1Δ₁₋₅₉.

5. A method to identify substances which affect the serine protease activity of LOP1Δ₁₋₅₉, comprising the steps:
a) Incubating LOP1Δ₁₋₅₉ with a target protein comprising at least one serine protease target site, in the presence and absence of a substance to be tested;
b) Monitoring the cleavage of said target protein;
c) Selecting substances which when present in step b) decrease the cleavage of said target protein.

6. The method of claim 5 wherein said target protein is a FtsZ polymer.

7. An inhibitor of the activity or expression of LOP1 or an active derivative thereof selected from the group antibodies, aptamers, antisense RNA or antisense DNA molecules or ribozymes.


SEQUENCE LISTING

<110> Institut Pasteur
      Institut National de la Santé et de la Recherche Médicale (INSERM)

<120> Methods to obtain a novel class of gram negative bacteria
      antibiotics which target an unknown cell division associated
      protein LOP1

<130> F226 - 197 EP

<160> 27

<170> PatentIn version 3.4

<210> 1
<211> 44
<212> DNA
<213> Artificial

<220>
<223> NG1281 primer

<400> 1
caaggaataa caatactgca gggcaaagcg ttaccccaac atcg                44

<210> 2
<211> 41
<212> DNA
<213> Artificial

<220>
<223> NG1282 primer

<400> 2
ttgtgatttg tggggatcct taacccgcca aatgctcgcg c                    41

<210> 3
<211> 35
<212> DNA
<213> Artificial

<220>
<223> SG127 primer

<400> 3
gcaacgccgg atcctggcgt agtttacgat tacca                           35

<210> 4
<211> 37
<212> DNA
<213> Artificial

<220>
<223> SG128 primer

<400> 4
gtgatttgtg gcagcatatg acccgccaaa tgctcgc                         37

<210>  5
<211>  41
<212>  DNA
<213>  Artificial

<220>
<223>  SG114 primer*

<400>  5
gtggggcggt gtaggacgcg gggcaacctg gctgatggac c                          41


<210>  6
<211>  41
<212>  DNA
<213>  Artificial

<220>
<223>  SG115 primer

<400>  6
ggtccatcag ccaggttgcc ccgcgtccta caccgcccca c                          41


<210>  7
<211>  40
<212>  DNA
<213>  Artificial

<220>
<223>  SG90 primer

<400>  7
ttcaaggaat aacaataaga ccatggaaag cgttacccca                            40


<210>  8
<211>  39
<212>  DNA
<213>  Artificial

<220>
<223>  SG91 primer

<400>  8
gtgatttgtg gcaggttgga tcccgccaaa tgctcgcgc                             39


<210>  9
<211>  40
<212>  DNA
<213>  Artificial

<220>
<223>  SG157 primer

<400>  9
gggctaatgg cgcgggtcgg taccatgggg ggtaaacgcg                            40


<210>  10
<211>  49
<212>  DNA

<213>  Artificial

<220>
<223>  SG169 primer

<400>  10
ggcgtatgct ttgtgtcttc gcgtttggat cccagcttac cgacccgcg                49


<210>  11
<211>  23
<212>  DNA
<213>  Artificial

<220>
<223>  SG150 primer

<400>  11
cccgggatcc accggtcgcc acc                                            23


<210>  12
<211>  26
<212>  DNA
<213>  Artificial

<220>
<223>  SG151 primer

<400>  12
gattatgatc tgaattcgcg gccgct                                         26


<210>  13
<211>  30
<212>  DNA
<213>  Artificial

<220>
<223>  SG219 primer

<400>  13
cgcgccagta cgaagcttgc cggatgcgcc                                     30


<210>  14
<211>  32
<212>  DNA
<213>  Artificial

<220>
<223>  SG155 primer

<400>  14
gtggcagttc tagacccgcc aaatgctcgc gc                                  32


<210>  15
<211>  53
<212>  DNA
<213>  Artificial

<220>

<223>  SG164 primer

<400>  15
ttattcaagg aataacaata agatcatgtg gggtaaacgc gaagacacaa agc          53


<210>  16
<211>  53
<212>  DNA
<213>  Artificial

<220>
<223>  SG165 primer

<400>  16
gctttgtgtc ttcgcgttta ccccacatga tcttattgtt attccttgaa taa          53


<210>  17
<211>  32
<212>  DNA
<213>  Artificial

<220>
<223>  SG278 primer

<400>  17
ggatccatgc aaagcgttac cccaacatcg ca                                 32


<210>  18
<211>  34
<212>  DNA
<213>  Artificial

<220>
<223>  SG328 primer

<400>  18
ggatccatgt ggggtaaacg cgaagacaca aagc                               34


<210>  19
<211>  34
<212>  DNA
<213>  Artificial

<220>
<223>  SG329 primer

<400>  19
gaattcttag ctacttgtac agctcgtcca tgcc                               34


<210>  20
<211>  21
<212>  DNA
<213>  Artificial

<220>
<223>  NWpr23 primer

<400>  20

atgttcatga cctgggaata t                                                21

<210>  21
<211>  21
<212>  DNA
<213>  Artificial

<220>
<223>  NWpr24 primer

<400>  21
gtcgcgcttc gcgccagtac g                                                21

<210>  22
<211>  20
<212>  DNA
<213>  Artificial

<220>
<223>  NWpr40 primer

<400>  22
acagcgtagt aaaagagacc                                                  20

<210>  23
<211>  20
<212>  DNA
<213>  Artificial

<220>
<223>  NWpr41 primer

<400>  23
cggaaacaat gccagaggtg                                                  20

<210>  24
<211>  1128
<212>  DNA
<213>  Escherichia coli

<400>  24
   atgcaaagcg ttaccccaac atcgcaatac ctgaaggcgc ttaatgaagg cagccatcaa      60
   cccgacgacg ttcaaaaaga ggccgtcagc cgcctggaaa ttatttatca ggaactcatc     120
   aatagcacgc caccagcccc caggacgagt gggctaatgg cgcgggtcgg taagctgtgg     180
   ggtaaacgcg aagacacaaa gcatacgcca gtgcgtggct tatatatgtg gggcggtgta     240
   ggacgcggga aaacctggct gatggacctt ttctatcaaa gcctgccggg agagcggaaa     300
   cagcgcctgc actttcaccg ttttatgctg cgggtgcatg aagagctaac tgccttacag     360
   gggcagaccg atccgctgga aattattgcc gatcgcttta aagccgaaac tgacgtgctc     420
   tgttttgacg aattttttgt ttctgatatt accgatgcca tgctacttgg cggtctgatg     480
   aaagccctgt tcgctcgcgg tattaccctg gtagcgacgt caaatattcc gccggatgaa     540
   ctttatcgaa atggcctgca acgtgcgcgt tttctgcctg caatcgatgc cattaaacag 600
   cattgtgatg taatgaacgt ggacgctggt gttgattatc gtctgcgtac actcactcag 660
   gcgcatctgt ggctttcgcc acttcacgat gaaacccggg cgcaaatgga taaactatgg 720
   ttggcgctgg cgggggggaa acgagaaaat tcaccgacgt tagaaatcaa ccatcggcca 780
   ttagcaacaa tgggcgtcga gaaccagacg ctggcggtct cttttactac gctgtgcgtc 840
   gacgcccgca gtcagcatga ctatattgcg ctctcacgtc tctttcatac ggtcatgttg 900
   tttgatgtac cagttatgac gcggttgatg gagagcgaag cgcggcgctt tattgcgctg 960
   gtggatgagt tttacgagcg ccatgtcaaa ttagtggtga gtgcagaagt gccgctgtat 1020
   gaaatttatc agggcgatcg gctgaagttt gagttccagc gttgcctgtc acgtctgcaa 1080
   gagatgcaaa gcgaagagta tctgaagcgc gagcatttgg cgggttaa 1128

<210> 25
<211> 375
<212> PRT
<213> Escherichia coli

<400> 25

Met Gln Ser Val Thr Pro Thr Ser Gln Tyr Leu Lys Ala Leu Asn Glu
1               5                   10                  15

Gly Ser His Gln Pro Asp Asp Val Gln Lys Glu Ala Val Ser Arg Leu
            20                  25                  30

Glu Ile Ile Tyr Gln Glu Leu Ile Asn Ser Thr Pro Pro Ala Pro Arg
        35                  40                  45

Thr Ser Gly Leu Met Ala Arg Val Gly Lys Leu Trp Gly Lys Arg Glu
    50                  55                  60

Asp Thr Lys His Thr Pro Val Arg Gly Leu Tyr Met Trp Gly Gly Val
65                  70                  75                  80

Gly Arg Gly Lys Thr Trp Leu Met Asp Leu Phe Tyr Gln Ser Leu Pro
                85                  90                  95

Gly Glu Arg Lys Gln Arg Leu His Phe His Arg Phe Met Leu Arg Val
            100                 105                 110

His Glu Glu Leu Thr Ala Leu Gln Gly Gln Thr Asp Pro Leu Glu Ile
        115                 120                 125

Ile Ala Asp Arg Phe Lys Ala Glu Thr Asp Val Leu Cys Phe Asp Glu
    130                 135                 140

Phe Phe Val Ser Asp Ile Thr Asp Ala Met Leu Leu Gly Gly Leu Met
145                 150                 155                 160

Lys Ala Leu Phe Ala Arg Gly Ile Thr Leu Val Ala Thr Ser Asn Ile
                165                 170                 175

Pro Pro Asp Glu Leu Tyr Arg Asn Gly Leu Gln Arg Ala Arg Phe Leu
            180                 185                 190

Pro Ala Ile Asp Ala Ile Lys Gln His Cys Asp Val Met Asn Val Asp
        195                 200                 205

Ala Gly Val Asp Tyr Arg Leu Arg Thr Leu Thr Gln Ala His Leu Trp
    210                 215                 220

Leu Ser Pro Leu His Asp Glu Thr Arg Ala Gln Met Asp Lys Leu Trp
225                 230                 235                 240

Leu Ala Leu Ala Gly Gly Lys Arg Glu Asn Ser Pro Thr Leu Glu Ile
                245                 250                 255

Asn His Arg Pro Leu Ala Thr Met Gly Val Glu Asn Gln Thr Leu Ala
            260                 265                 270

Val Ser Phe Thr Thr Leu Cys Val Asp Ala Arg Ser Gln His Asp Tyr
        275                 280                 285

Ile Ala Leu Ser Arg Leu Phe His Thr Val Met Leu Phe Asp Val Pro
    290                 295                 300

Val Met Thr Arg Leu Met Glu Ser Glu Ala Arg Arg Phe Ile Ala Leu
305                 310                 315                 320

Val Asp Glu Phe Tyr Glu Arg His Val Lys Leu Val Val Ser Ala Glu
                325                 330                 335

Val Pro Leu Tyr Glu Ile Tyr Gln Gly Asp Arg Leu Lys Phe Glu Phe
            340                 345                 350

Gln Arg Cys Leu Ser Arg Leu Gln Glu Met Gln Ser Glu Glu Tyr Leu
        355                 360                 365

Lys Arg Glu His Leu Ala Gly
    370                 375

<210>  26
<211>  59

<212>  PRT
<213>  Escherichia coli

<400>  26

Met Gln Ser Val Thr Pro Thr Ser Gln Tyr Leu Lys Ala Leu Asn Glu
1               5                   10                  15

Gly Ser His Gln Pro Asp Asp Val Gln Lys Glu Ala Val Ser Arg Leu
            20                  25                  30

Glu Ile Ile Tyr Gln Glu Leu Ile Asn Ser Thr Pro Pro Ala Pro Arg
        35                  40                  45

Thr Ser Gly Leu Met Ala Arg Val Gly Lys Leu
    50                  55

<210>  27
<211>  34
<212>  DNA
<213>  Artificial

<220>
<223>  SG154 primer

<400>  27
tactgcaacg cctgaagctt gcgtagttta cgat                                34